# DEMANDE DE BREVET EUROPEEN

(11) **EP 2 764 893 A1**
(43) Date de publication de la demande: **13.08.2014**
(21) Numéro de dépôt: 14151601.3
(22) Date de dépôt: 17.01.2014
(51) Int. Cl.: A61Q 7/00, A61Q 19/00, A61Q 19/08, A61K 8/97, A61Q 5/00, A61K 8/92

(54) **Composition cosmétique ou dermatologique comprenant au moins deux extraits d'angélique et utilisations**

(30) Priorité: 11.02.2013 FR 1351148; 25.02.2013 FR 1351629
(71) Demandeur: Laboratoires M & L, 04100 Manosque En Provence (FR)
(72) Inventeur: Devilard, Elisabeth, 04130 VOLX (FR); Tourel, Cécile, 13100 AIX-EN-PROVENCE (FR); Chalmeton, Gaëlle, 07460 BERRIAS ET CASTELJAU (FR)
(74) Mandataire: Novagraaf Technologies

(57) **Abrégé**

La présente invention se rapporte à une composition cosmétique ou dermatologique, ainsi qu'à une trousse de soin comprenant la composition de l'invention. La présente invention se rapporte également à une composition cosmétique comprenant au moins un extrait de racines d'angélique et un extrait de graines d'angélique et son utilisation dans un traitement cosmétique.

SEQUENCE LISTING

<110>  Laboratoires M&L

<120>  COMPOSITION COSMETIQUE OU DERMATOLOGIQUE COMPRENANT AU MOINS UN
       EXTRAIT D'ANGELIQUE ET UTILISATIONS

<130>  BNT216297EP00

<150>  FR1351148
<151>  2013-02-11

<150>  FR1351629
<151>  2013-02-11

<160>  14

<170>  PatentIn version 3.5

<210>  1
<211>  22
<212>  DNA
<213>  Artificial Sequence

<220>
<223>  amorce sens Lum L

<400>  1
tggaggtcaa tcaacttgag aa                                                22


<210>  2
<211>  21
<212>  DNA
<213>  Artificial Sequence

<220>
<223>  amorce anti-sens Lum R

<400>  2
caaacgcaaa tgcttgatct t                                                 21


<210>  3
<211>  19
<212>  DNA
<213>  Artificial Sequence

<220>
<223>  amorce sens VCAN L

<400>  3
gcacctgtgt gccaggata                                                    19


<210>  4
<211>  24
<212>  DNA
<213>  Artificial Sequence

<220>
<223>  amorce anti-sens Vcan R

<400>  4
cagggattag agtgacattc atca                                              24


<210>  5
<211>  19
<212>  DNA
<213>  Artificial Sequence

<220>
<223>  amorce sens Gapdh L

<400>  5
agccacatcg ctcagacac                                                    19


<210>  6
<211>  19
<212>  DNA
<213>  Artificial Sequence

<220>
<223>  amorce anti-sens Gapdh R

<400>  6
gcccaatacg accaaatcc                                                    19


<210>  7
<211>  24
<212>  DNA
<213>  Artificial Sequence

<220>
<223>  amorce sens MMP 3 L

<400>  7
cagggattag agtgacattc atca                                              24


<210>  8
<211>  21
<212>  DNA
<213>  Artificial Sequence

<220>
<223>  amorce anti-sens MMP3 R

<400>  8
ttcagctatt tgcttgggaa a                                                 21


<210>  9
<211>  19
<212>  DNA
<213>  Artificial Sequence

<220>
<223>  amorce sens LOX L

<400>  9
tgggaatggc acagttgtc                                                    19

<210>  10
<211>  20
<212>  DNA
<213>  Artificial Sequence

<220>
<223>  amorce anti-sens LOX R

<400>  10
aaacttgctt tgtggccttc                                                    20


<210>  11
<211>  20
<212>  DNA
<213>  Artificial Sequence

<220>
<223>  amorce sens Krt34 L

<400>  11
ggagagtgag gactgcaagc                                                    20


<210>  12
<211>  20
<212>  DNA
<213>  Artificial Sequence

<220>
<223>  amorce anti-sens Krt34 R

<400>  12
ccacaggagt tgccactagc                                                    20


<210>  13
<211>  20
<212>  DNA
<213>  Artificial Sequence

<220>
<223>  amorce sens Rptn L

<400>  13
gctcttggct gagtttggag                                                    20


<210>  14
<211>  21
<212>  DNA
<213>  Artificial Sequence

<220>
<223>  amorce anti-sens Rptn R

<400>  14
aggttcaaga tggtttccac a                                                  21

## Description

### Domaine technique

La présente invention se rapporte à une composition cosmétique ou dermatologique, ainsi qu'à une trousse de soin comprenant la composition de l'invention.

En particulier, la présente invention se rapporte à une composition cosmétique comprenant au moins un extrait de racines d'angélique et un extrait de graines d'angélique.

La présente invention se rapporte également à une composition cosmétique hydratante antiâge comprenant au moins un extrait de racines d'angélique et un extrait de graines d'angélique.

La présente invention se rapporte en outre à une composition cosmétique capillaire réparatrice comprenant au moins un extrait de racines d'angélique et un extrait de graines d'angélique.

La présente invention se rapporte également à l'utilisation d'une composition cosmétique comprenant au moins un extrait de racines d'angélique et un extrait de graines d'angélique.

La présente invention se rapporte notamment à un procédé cosmétique comprenant l'utilisation d'une composition cosmétique comprenant au moins un extrait de racines d'angélique et un extrait de graines d'angélique.

La présente invention trouve une application notamment dans les domaines cosmétique et dermatologique.

Dans la description ci-dessous, les références entre crochets ([]) renvoient à la liste des références présentées à la fin du texte.

### Etat de la technique

Plus qu'une enveloppe extérieure, la peau est un organe à part entière qui joue un rôle essentiel, non seulement dans la protection du corps contre les agressions extérieures, mais aussi sur le plan esthétique et émotionnel. Une peau en bonne santé est une peau bien hydratée. L'eau est nécessaire au bon fonctionnement des cellules cutanées et participe à son renouvellement. L'eau est apportée en permanence à la peau par la circulation sanguine, au niveau du derme, puis par diffusion vers l'épiderme. En revanche, la peau est incapable de puiser l'eau du milieu extérieur. Ce n'est que dans les conditions extrêmes (hammam, bain prolongé) qu'une petite quantité d'eau peut pénétrer dans les couches superficielles en voie de desquamation.

Parallèlement, la peau perd continuellement de l'eau :
- par transpiration: cette eau provient des glandes sudoripares situées dans le derme.
- par évaporation: cette eau provient de la diffusion à travers l'épiderme.

L'hydratation cutanée dépend donc de l'équilibre entre les pertes et les apports en eau. La peau, au niveau de l'épiderme, dispose de moyens naturels pour assurer cet équilibre (le ciment lipidique, le MNF, la stimulation des aquaporines).

Au niveau du derme, constitué de fibroblastes dispersés dans la matrice extracellulaire, l'eau et les nutriments sont apportés par les capillaires sanguins. La constitution particulière de cette matrice extracellulaire assure le maintien d'une bonne hydratation du derme, la teneur en eau totale du derme étant équivalente à celle de l'épiderme. Le derme est constitué par 70% de fibres de collagènes, fibrillines, élastine et par des glycosaminoglycanes désignés depuis longtemps sous le terme de "mucopolysaccharides acides" en raison de leur forte capacité de rétention de l'eau, de leur nature glucidique et de leur caractère acide provenant de leurs multiples charges négatives. La combinaison d'une protéine et d'un glycosaminoglycane constitue un protéoglycane qui présente des propriétés de liaison aux photons de l'eau (pièges à eau) de part la longueur des chaines constituées de sucres non ramifiées et O-glycosilées. Les protéoglycanes sont les composants essentiels de la matrice extracellulaire. Certains sont sécrétés (aggrecan, biglycan, decorine, versican...) et d'autres restent attachés à la membrane (biglycan, syndecan....). De ce fait, il en résulte que la teneur en eau liée du derme est deux fois plus importante que celle de l'épiderme [1].

Il existe dans l'état de la technique de nombreuses compositions hydratantes et/ou anti-âges, notamment des compositions qui permettent de faire des « films » à la surface de la peau permettant ainsi de diminuer la perte de l'eau. Toutefois, ces compositions permettent uniquement une hydratation superficielle de la peau et ne permettent pas une efficacité hydratante et anti-âge systématique et prononcée.

Il existe donc un réel besoin dans l'état de la technique de trouver une composition permettant à la fois une hydratation cutanée superficielle et une hydratation « en profondeur » de la peau.

Par ailleurs, les cheveux font partie d'un système complexe appelé follicule pilo-sébacé. Ils se composent de kératine (95%), de graisses (triglycérides, cire, cholestérol, phospholipides, scalène), de minéraux (fer, magnésium, zinc, cuivre, plomb), d'eau et de mélanine (substances colorées, eumélanine dans les cheveux bruns et phéomélanine dans les cheveux roux, blonds ou châtains dorés). Dans les cheveux, on peut distinguer la partie visible externe du follicule pilifère appelée tige, la partie interne appelée racine, la partie profonde interne du follicule appelée bulbe où la matrice se trouve à la base. Les cellules de la matrice possèdent un noyau vésiculeux avec un cytoplasme dont l'indice mitotique est très élevé et dont l'équivalent existe uniquement dans l'activité multiplicative de la moelle osseuse. Associées à d'autres protéines, les kératines se structurent en proto puis microfibrilles. Celles-ci sont enserrées dans une enveloppe externe, formée d'écailles de kératine et sont très rigides du fait de la présence de ponts disulfures entre les protéines de kératine. Les kératines et autres protéines associées aux kératines confèrent aux cheveux la résistance mécanique et l'élasticité relative des cheveux.

Il existe dans l'état de la technique de nombreuses compositions afin d'améliorer l'aspect et la résistance des cheveux, en particulier des compositions gainantes, par exemple des masques, des baumes, permettant d'améliorer l'aspect du cheveu. Toutefois, ces compositions ne permettent pas de traiter et/ou de réparer efficacement et durablement un cheveu abîmé. En particulier, ces compositions ne permettent pas une réparation/traitement des altérations de la fibre capillaire et son renforcement.

Il existe donc un réel besoin de trouver un nouveau composé, composition et/ou un moyen de traitement efficace pour améliorer l'apparence des fibres capillaires palliant l'ensemble des défauts, inconvénients et obstacles de l'art antérieur cités ci-dessus.

### Exposé de l'invention

La présente invention a précisément pour but de répondre à ces besoins et inconvénients en fournissant une composition cosmétique ou dermatologique comprenant au moins un extrait de racines d'angélique et un extrait de graines d'angélique. La présente invention se rapporte également à l'utilisation cosmétique et/ou dermatologique de cette composition.

La présente invention se rapporte également à l'utilisation d'au moins un extrait de racines d'angélique et d'au moins un extrait de graines d'angélique comme actif cosmétique.

En particulier, la présente invention se rapporte à l'utilisation cosmétique et/ou dermatologique d'une composition cosmétique ou dermatologique comprenant au moins un extrait de racines d'angélique et un extrait de graines d'angélique en tant que principe actif cosmétique hydratant cutané.

Les inventeurs de la présente sont en effet les tout premiers à avoir mis en évidence, de manière tout à fait inattendue, qu'une composition comprenant au moins un extrait de racines d'Angélique et un extrait de graines d'Angélique permet d'hydrater efficacement et durablement la peau et de réparer les cheveux. En particulier, les inventeurs ont mis en évidence que la composition de l'invention permet avantageusement d'hydrater la peau en profondeur et notamment d'hydrater le derme.

La présente invention se rapporte à l'utilisation cosmétique et/ou dermatologique d'une composition cosmétique ou dermatologique comprenant au moins un extrait de racines d'angélique et un extrait de graines d'angélique en tant que principe actif cosmétique pour la réparation capillaire.

Les inventeurs ont également démontré de manière surprenante et inattendue que la composition de l'invention permet également, de part son effet hydratant, de renforcer, reconstituer les fibres capillaires. En outre, les inventeurs ont démontré que la composition selon l'invention permet avantageusement de stimuler la pousse et la micro circulation au niveau du cuir chevelu.

Dans la présente par « composition cosmétique » on entend toute composition utilisable dans le domaine cosmétique destiné à être mis en contact avec les parties superficielles du corps humain, par exemple la peau, les cheveux, les ongles..

Dans la présente par « graines », on entend le petit organe des plantes, enfermé dans leur fruit et qui permet leur reproduction.

Dans la présente par « angélique », on entend une plante de la famille des Apiacées. Il peut s'agir, par exemple de l'angélique officinale ou *Angelica archangelica,* également nommée angélique vraie, Archangélique.

Dans la présente par « graines d'angélique », on entend toutes graines obtenues à partir d'angélique. Il peut s'agir, par exemple, de graines obtenues de l'angélique officinale ou *Angelica archangelica.* Il peut s'agir par exemple de toutes graines d'angéliques disponibles dans le commerce.

Dans la présente, par « extrait de graines », on entend tout ou partie d'une graine, sous forme brute ou ayant subi un traitement. Le traitement peut consister, par exemple, en un nettoyage et/ou un broyage et/ou un séchage et/ou une macération et/ou une purification d'une graine, par exemple par distillation et/ou par extraction liquide/liquide et/ou par extraction liquide/solide et/ou par extraction assistée par hyperfréquence et/ou par ultrasons.

Selon l'invention, l'extrait de graines peut se trouver sous la forme de fragments ou de poudre de graines, ou encore sous forme d'une solution qui peut être une solution hydrosoluble, liposoluble.

Selon l'invention, lorsque l'extrait est un extrait hydrosoluble, il peut s'agir, par exemple, d'un extrait aqueux, d'un extrait hydroglycolique, d'un extrait hydro-alcoolique, hydro-glycériné, hydro-alcoolique glycériné, d'un extrait obtenu par distillation, par macération, par extraction assistée par hyperfréquences ou par ultrasons, par exemple un extrait obtenu selon le procédé décrit dans Farid Chemat : Eco-extraction du végétal, Editions Dunod, 2011 [3].

Selon l'invention, lorsque l'extrait de graines d'angélique est un extrait hydroglycolique, il peut s'agir par exemple d'un extrait disponible dans le commerce, par exemple l'Angelica Phytelene EG510 de Greentech.

Selon l'invention, lorsque l'extrait de graines d'angélique est un extrait hydro-alcoolique glycériné, il peut s'agir par exemple d'un extrait hydro-alcoolique glycériné obtenu à partir d'un procédé comprenant les étapes de lavage et séchage des graines, broyage des graines, trempage des graines broyées dans une solution hydroalcoolique 50/50 à un ratio de 10%, puis extraction sous ultra-sons, pendant, par exemple de 1 à 120 minutes, par exemple 90 minutes, à une température comprise de 30 à 60°C, par exemple à 50°C, traitement au charbon actif, filtration de la solution obtenue et dilution de la solution à 100% dans de la glycérine.

Selon l'invention, lorsque l'extrait de graines d'angélique est un extrait liposoluble, il peut s'agir d'un extrait huileux de graines d'angélique obtenu par tout procédé connu de l'homme du métier, par exemple, il peut s'agir d'un extrait obtenu par macération dans un solvant huileux, par extraction assistée par hyperfréquences ou par ultra-sons, par extraction super critique, (Farid Chemat: Eco-extraction du végétal, Editions Dunod, 2011 [3]) Il peut s'agir, par exemple d'une huile essentielle de graines d'Angélique obtenue par tout procédé connu de l'homme du métier, par exemple par distillation à la vapeur d'eau, et/ou une huile essentielle de graines d'angélique disponible dans le commerce, par exemple une huile essentielle commercialisé par la société In Lustrys (France).

Selon l'invention, l'extrait de graines d'angélique peut être présent dans la composition à une concentration comprise de 0,001 % à 10% en poids par rapport au poids total de la composition, par exemple de 0,01 % à 5% en poids par rapport au poids total de ladite composition.

Avantageusement selon l'invention, l'extrait hydro-alcoolique glycériné de graines d'angélique permet d'augmenter la production de protéoglycanes de la matrice extracellulaire dermique captant ainsi plus d'eau et hydratant durablement le derme.

Dans la présente par « racines d'angélique » on entend toutes racines obtenues à partir d'angélique. Il peut s'agir, par exemple, de racines obtenues de l'angélique officinale ou *Angelica archangelica.* Il peut s'agir par exemple de toutes racines d'angéliques disponibles dans le commerce.

Selon l'invention, par « extrait de racines » on entend tout ou partie d'une racine, sous forme brute ou ayant subi un traitement. Le traitement peut consister, par exemple, en un nettoyage et/ou un broyage et/ou un séchage et/ou une macération d'une racine, par exemple par distillation et/ou par extraction liquide/solide et/ou par extraction assistée par hyperfréquence et/ou par ultrasons.

Selon l'invention, l'extrait de racines d'angélique peut être un extrait aqueux, un extrait huileux, une huile essentielle ou un mélange de ceux-ci.

Selon l'invention, lorsque l'extrait de racines d'angélique est un extrait aqueux, il peut s'agir, par exemple, d'un extrait obtenu par extraction aqueuse, par exemple une eau d'angélique, par exemple Végébios d'angélique (marque de commerce) commercialisée par la société Solabia (France).

Selon l'invention, lorsque l'extrait de racines est un extrait huileux, il peut s'agir d'une huile essentielle, il peut s'agir d'une huile essentielle obtenue par tout procédé connu de l'homme du métier, par exemple, une huile essentielle obtenus par obtenue par distillation à la vapeur d'eau et/ou une huile essentielle de racines d'angélique disponible dans le commerce, par exemple, une huile essentielle d'angélique commercialisée par les Laboratoires Rosier Davenne (France)

Selon l'invention, l'extrait de racines d'angélique peut être présent dans la composition à une concentration comprise de 0,001% à 10% en poids par rapport au poids total de la composition, par exemple de 0,01 % à 5% en poids par rapport au poids total de ladite composition.

Selon l'invention, l'extrait aqueux de racines d'Angélique peut être présent dans la composition par exemple à une concentration de 0,01 à 10% en poids par rapport au poids total de ladite composition, par exemple de 0,01 à 5% en poids par rapport au poids total de ladite composition.

Avantageusement, l'extrait aqueux de racines d'angélique permet notamment de stimuler les aquaporines, en particulier d'améliorer la circulation de l'eau vers les cellules de la couche cornée, et favoriser le renouvellement cellulaire.

Selon l'invention, l'extrait huileux de racines d'Angélique peut être présent dans la composition par exemple à une concentration de 0,001 à 0,8% en poids par rapport au poids total de ladite composition, par exemple de 0,001 à 0,5% en poids par rapport au poids total de ladite composition.

Avantageusement, l'extrait huileux de racines d'Angélique permet notamment de préserver l'élastine, de protéger des radicaux libres et de stimuler la synthèse des GAGs.

Selon l'invention, la composition cosmétique peut comprendre avantageusement, une huile essentielle de racines d'angélique, un extrait aqueux et/ou huileux de racines d'angéliques et un extrait de graines d'angélique.

Avantageusement, la composition cosmétique selon l'invention comprenant au moins un extrait de graines d'Angélique, une huile essentielle de racines d'angélique et/ou un extrait de racines d'Angélique permet d'hydrater durablement le derme et les couches profondes de la peau.

Selon l'invention, la composition cosmétique peut comprendre avantageusement une huile essentielle de racines d'angélique et une huile essentielle de graines d'angélique.

Avantageusement, la composition cosmétique selon l'invention comprenant une huile essentielle de racines d'angélique et une huile essentielle de graines d'angélique peut être particulièrement adaptée à une utilisation dans un traitement cosmétique capillaire.

Selon l'invention, la composition de la présente invention peut comprendre en outre un extrait d'avoine, notamment un extrait d*'Avena strigosa,* également nommé avoine noire.

Selon l'invention, l'extrait d'avoine peut être un extrait hydrosoluble, un extrait huileux, ou un mélange de ceux-ci. Il peut s'agir, par exemple d'un extrait disponible dans le commerce, par exemple un extrait commercialisé sous la référence Aquarich Eco par la société RAHN.

Selon l'invention, l'extrait d'avoine peut être présent dans la composition à une concentration comprise de 0,001% à 10% en poids par rapport au poids total de la composition, par exemple de 0,01% à 5% en poids par rapport au poids total de ladite composition.

Avantageusement, lorsque la composition comprend de l'extrait d'avoine, notamment l'extrait commercialisé sous la référence Aquarich Eco par la société RAHN, la composition selon l'invention permet, de par sa richesse en substances nutritives, d'améliorer la structure du cheveu. En outre, avantageusement, les acides aminés compris dans la composition selon l'invention peuvent adhérer et pénétrer dans les cheveux et aider à les réparer. De plus, la composition selon l'invention comprenant en outre de l'extrait d'avoine permet avantageusement de par la présence de polysaccharides de générer un film et de favoriser l'amélioration de la structure de la surface des cheveux, les rendant plus faciles à démêler et plus brillant.

Selon l'invention, quel que soit l'extrait ou le mélange d'extraits utilisés, la composition cosmétique ou dermatologique de la présente invention peut comprendre en outre au moins un agent actif additionnel choisi, par exemple parmi un agent anti-âge, un agent hydratant, un agent apaisant, un agent agissant sur la micro-circulation, un agent raffermissant, un agent tenseur ou un mélange de ces agents. Ces agents peuvent être ceux couramment utilisés dans les produits cosmétiques et/ou dermatologiques. Des exemples d'agents anti-âges utilisables peuvent être par exemple le silicium ou la vitamine C. Des exemples d'agents hydratants utilisables peuvent être par exemple la glycérine, l'acide hyaluronique ou le D-Panthenol. Des exemples d'agents apaisants utilisables peuvent être par exemple le bisabolol ou l'allantoïne. Des exemples d'agents agissant sur la micro-circulation peuvent être par exemple de l'escine. Des exemples d'agents raffermissants peuvent être par exemple un dérivé de silicium, la caféine, des agents tenseurs par exemple des protéines d'amande. Ce ou ces agent(s) peuvent être utilisé(s) dans la composition de la présente invention par exemple aux concentrations usuellement utilisées et connues de l'homme du métier dans les compositions cosmétiques ou dermatologiques. La composition cosmétique ou dermatologique de la présente invention peut également comprendre en outre au moins un agent actif additionnel choisi, par exemple parmi un agent conditionneur, un agent réparateur. Ces agents peuvent être ceux couramment utilisés dans les produits cosmétiques et/ou dermatologiques. Des exemples d'agents conditionneurs utilisables peuvent être par exemple les protéines de maïs, de riz, les quaterniums, des protéines hydrolysées quaternisées. Des agents réparateurs peuvent être par exemple des céramides, des huiles végétales d'amande, d'argan, du beurre de karité.

La composition cosmétique ou dermatologique selon l'invention peut comprendre un ou plusieurs supports cosmétiquement acceptable. Dans la présente, par « support cosmétiquement acceptable» on entend tout support cosmétique connue de l'homme du métier, il peut s'agir par exemple de tout support cosmétique pouvant être cité dans le dictionnaire INCI (International Nomenclature of Cosmetic Ingredients) publié par le PCPC (Personal Care Products council).

La composition cosmétique ou dermatologique selon l'invention peut comprendre un ou plusieurs adjuvants connus de l'homme du métier. Il peut s'agir par exemple d'un ou plusieurs adjuvant(s) choisi(s) parmi des agents de type esters, des agents hydratants, des agents émollients, des agents épaississants minéraux, des agents épaississants organiques, associatifs ou non, des filtres solaires organiques hydrosolubles et liposolubles, des filtres solaires minéraux, des composés siliconés, des parfums, des conservateurs, des céramides, et pseudo-céramides, des vitamines et les provitamines, des protéines, des agents séquestrants, des agents alcanisants, des agents acidifiants, des agents réducteurs, des agents oxydants, des charges minérales, des colorants ou tout autre adjuvant pouvant être cité dans le dictionnaire INCI (International Nomenclature of Cosmetic Ingredients) publié par le PCPC (Personal Care Products council).

Selon l'invention, la composition cosmétique ou dermatologique peut, par exemple, se présenter sous toute forme connue de l'homme du métier. Il peut s'agir, par exemple d'une émulsion huile-dans-l'eau, eau-dans-l'huile, eau dans silicone, d'une émulsion multiple, d'une microémulsion, d'une nano-émulsion, d'une émulsion solide, d'un gel aqueux ou hydro-alcoolique, d'une crème, d'un lait, d'une lotion, d'une pommade, d'une huile, d'un baume, d'un onguent, d'un masque, d'une poudre, d'un support imbibé, par exemple un patch transdermique, d'une lotion aqueuse ou hydroalcoolique et/ou une cire, d'un produit de maquillage, par exemple un fond de teint, d'un shampooing, d'un après-shampooing, d'un masque, sérum, lotion capillaire.

Selon l'invention, la composition cosmétique ou dermatologique peut être sous toute forme connue de l'homme du métier dans les domaines de la cosmétique et de la dermatologie, sans aucune restriction galénique particulière.

La composition cosmétique ou dermatologique selon la présente invention peut être, par exemple une composition pour le soin du visage, du corps, des cheveux, par exemple des compositions pour le visage et/ou le corps et/ou les cheveux.

La composition cosmétique ou dermatologique de l'invention peut être obtenue par tout procédé approprié connu de l'homme du métier pour la fabrication d'une composition cosmétique et/ou dermatologique. Il peut s'agir, par exemple d'un simple mélange. Il peut s'agir d'un mélange de matières tensioactives dans de l'eau. Il peut s'agir également, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'Inversion de Phase (TIP), procédé classiquement utilisé par l'homme de l'art pour obtenir des émulsions huile dans eau dont les gouttelettes dispersées sont particulièrement fines, par exemple avec un diamètre de 0,1 à 1 µm.

Selon l'invention, la composition cosmétique peut être une composition utilisée dans un soin cosmétique, par exemple un soin cosmétique ou dans un traitement cosmétique pour hydrater la peau et/ou pour renforcer et/ou reconstituer les fibres capillaires et/ou stimuler la pousse des cheveux.

Dans la présente par « soin cosmétique » on entend par exemple l'application cutanée et/ou sur les cheveux d'une composition selon l'invention.

Selon l'invention, la composition cosmétique peut être utilisée dans un traitement cosmétique anti-âge et/ou hydratant. Il peut s'agir par exemple d'une composition cosmétique pour le traitement du vieillissement cutané, par exemple des rides et ridules, par exemple en hydratant durablement le tissu cutané redonnant du volume à la peau. Il peut s'agir par exemple d'une composition cosmétique pour le traitement de la déshydratation, par exemple une composition cosmétique réhydratante. Avantageusement, la composition de l'invention a une action de par son action hydratante en profondeur réhydratant le tissu cutané.

Avantageusement, la composition selon l'invention permet, lors de l'utilisation dans un traitement cosmétique hydratant de favoriser le captage et la fixation d'un plus grand nombre de molécules d'eau à travers le derme et les couches profondes de l'épiderme.

Selon l'invention, la composition cosmétique selon l'invention peut être utilisée dans un traitement réparateur pour les cheveux. En particulier, la composition de l'invention permet avantageusement de renforcer ainsi que de reconstituer les fibres capillaires et stimuler la pousse et la micro circulation au niveau du cuir chevelu.

Avantageusement, la composition cosmétique selon l'invention permet également de protéger le cheveu, la fibre capillaire des agressions extérieures, par exemple des rayonnements Ultra-Violet, de la chaleur, par exemple la chaleur fournie par un sèche cheveux, et/ou des agressions mécaniques, par exemple celles dues au brossage des cheveux.

La présente invention a également pour objet un procédé de traitement cosmétique non thérapeutique comprenant l'application sur la peau et/ou les cheveux d'une composition cosmétique selon l'invention. La présente invention a également pour objet un procédé de traitement cosmétique comprenant l'application sur la peau d'une composition cosmétique comprenant au moins un extrait de graines d'Angéliques et au moins un extrait de racines d'Angélique. Avantageusement, le procédé de traitement cosmétique comprend l'application d'une composition cosmétique comprenant au moins un extrait de graines d'Angéliques et au moins un extrait de racines d'Angélique et/ou une huile essentielle de racines d'Angélique.

L'extrait de graines d'Angélique, l'extrait de racines d'Angélique sont tels que définis ci-dessus.

La composition cosmétique peut être celle définie précédemment.

La présente invention a également pour objet un procédé de traitement cosmétique comprenant l'application sur la peau d'une composition cosmétique selon l'invention.

Selon l'invention, le traitement cosmétique peut-être un traitement hydratant de la peau, un traitement réparateur capillaire et/ou un traitement cosmétique de la chute des cheveux.

Dans la présente par traitement cosmétique on entend un traitement cosmétique non thérapeutique.

Pour cette application, on peut, par exemple, utiliser les formes galéniques décrites ci-dessus. L'application sur la peau et/ou sur les cheveux peut donc être réalisée en fonction de la forme galénique utilisée.

Il peut s'agir, par exemple d'une simple application sur la peau ou d'une application accompagnée d'un massage de la peau avec la composition de la présente invention.

L'application est de préférence réalisée avec une quantité suffisante de la composition afin que toute la surface de la peau à traiter soit traitée. Il peut s'agir par exemple d'une application classique, comme une crème sur la peau. Il peut s'agir par exemple aussi d'une application pour former un masque traitant.

Il peut s'agir également, par exemple d'une simple application sur les cheveux ou d'une application accompagnée d'un massage du cuir chevelu avec la composition de la présente invention.

L'application sur les cheveux est de préférence réalisée avec une quantité suffisante de la composition afin que tous les cheveux et/ou fibre capillaire à traiter soit traitée. Il peut s'agir par exemple d'une application classique, comme un champoing, et/ou une application multiple, par exemple un champoing, un après champoing et/ou un masque traitant. L'application peut être, par exemple une application quotidienne, hebdomadaire et bimensuelle. Il peut s'agir par exemple d'une application une fois par jour, deux fois par jour ou plus. Lorsque l'application est multiple, elle peut être, par exemple concomitante ou successive.

La présente invention se rapporte également à une trousse de soin cosmétique comprenant une composition cosmétique selon la présente invention et un manuel ou une notice d'utilisation. La trousse peut être par exemple un conditionnement.

Le manuel ou cette notice peut avoir notamment pour fonction d'expliquer à l'utilisateur la manière et la fréquence d'application sur la peau et ou sur les cheveux de la composition de la présente invention.

Selon l'invention, la trousse de soin cosmétique peut également comprendre un applicateur, par exemple une spatule, une brosse, un applicateur dynamique, par exemple un dispositif de massage imprégné de la composition cosmétique de l'invention.

Les inventeurs de la présente invention ont clairement démontré et de manière surprenante que la présente invention comprenant un extrait de graines d'Angélique et un extrait de racines d'Angélique permet avantageusement une action synergique sur l'hydratation de la peau. En particulier, les inventeurs sont en effet les tout premiers à avoir mis en évidence, de manière tout à fait inattendue, qu'une composition comprenant au moins un extrait de graines d'Angélique et un extrait de racines d'Angélique permet avantageusement et de manière synergique, d'hydrater durablement et efficacement la peau depuis le derme jusqu'à l'épiderme.

Les inventeurs de la présente invention ont en outre clairement démontré et de manière surprenante que la présente invention, comprenant un extrait de graines d'Angélique et un extrait de racines d'Angélique, permet avantageusement une action synergique anti-âge, notamment via l'inhibition métalloprotéinase (MMP), en particulier la MMP3 et 7, inhibant ainsi la rupture des fibres de collagènes et de l'élastine qui intervient avec le vieillissement cutané, les agressions extérieures, par exemple la pollution, les ultra-violets (UV), les stress, etc.

Les inventeurs de la présente invention ont en outre clairement démontré et de manière surprenante que la présente invention comprenant un extrait de graines d'Angélique et un extrait de racines d'Angélique permet avantageusement une action synergique anti-âge, notamment via la stimulation des gènes LOX, permettant ainsi d'obtenir une élastine fonctionnelle.

Ainsi, la présente invention permet une efficacité hydratante et anti-âge systématique et prononcée.

De plus, les inventeurs de la présente sont les tout premiers à avoir mis en évidence, de manière tout à fait inattendue, qu'une composition comprenant un extrait de graines d'angélique et un extrait de racines d'angélique permet également avantageusement de stimuler efficacement la pousse des cheveux, la microcirculation cutanée et de renforcer les fibres capillaires et/ou le traitement cosmétique de la chute des cheveux.

Ainsi, la composition selon l'invention permet de traiter et de réparer efficacement et durablement un cheveu abîmé, notamment par une réparation, un traitement des altérations de la fibre capillaire et son renforcement.

D'autres caractéristiques et avantages apparaîtront encore à l'homme du métier à la lecture des exemples ci-dessous, donnes à titre illustratif et non limitatif.

### Brève description des figures

- La figure 1 représente une photographie d'une fibre capillaire en microscopie électronique après application de compositions selon l'invention après application d'un procédé mécanique.
- La figure 2 représente une photographie d'une fibre capillaire en microscopie électronique après application d'une composition de l'état de la technique après application d'un procédé mécanique.

### EXEMPLES

### Exemple 1 : Exemple de composition cosmétique pour le soin du visage selon la présente invention.

Une composition de base décrite dans le tableau 1 ci-dessous a été préparée par mélange des différents composants indiqués dans le tableau 1 ci-dessous :

**Tableau 1 : composition de base pour le visage**

| **Ingrédients** | **Quantité en % poids par rapport au poids total de la composition (%)** |
|---|---|
| Alkyl polyglucoside | 2,5-5 |
| Caprylique caprique triglycérides | 5-10 |
| Beurre de karité | 1-4 |
| Glycérine | 3-8 |
| Gomme xanthane (gélifiant) | 0,1-0,5 |
| Carbomer (gélifiant) | 0,1-0,5 |
| Parfum | Qs |
| Conservateurs | Qs |
| Eau | qsp 100% |

La composition de base a été préparée selon le procédé suivant :
- la glycérine a été introduite dans l'eau et mélangée à 1500 t/min
- les gélifiants ont été dispersés sous agitation à 1500 t/min jusqu'à dissolution.
- la phase huileuse préalablement préparée par mélange, par exemple de l'émulsionnant, du beurre de karité et des caprylic/capric triglycérides jusqu'à obtention d'un mélange homogène a été introduite sous agitation à une vitesse de 1500 tours par minute
- le parfum et les conservateurs, par exemple l'éthylhexyl glycérine ont été ensuite ajoutés.

A partir de cette composition de base, pour le soin du visage, ont été fabriquées différentes compositions en ajoutant différents extraits en différentes quantités :
- des compositions comprenant de l'huile essentielle de racines d'angélique provenant des Laboratoires Rosier Davenne
- des compositions comprenant de l'extrait aqueux de racines d'angélique provenant de la société Solabia
- des compositions comprenant de l'extrait hydro-alcoolique glycériné de graines d'angélique provenant des Laboratoires M&L.

Les valeurs en parfum, conservateur et eau étant alors ajustées comme indiqué dans le tableau 1 ci-dessus.

Les pourcentages en poids des différents extraits de racines et graines d'angélique sont indiqués dans le tableau 2 suivant.

**Tableau 2: pourcentage en poids des extraits par rapport au poids de la composition**

| Compositions | % en poids d'extrait hydro-alcoolique glycériné de graines d'angélique par rapport au poids total de la composition (%) | % en poids d'extrait aqueux de racines d'angélique par rapport au poids total de la composition (%) | % en poids d'huile essentielle de racines d'angélique par rapport au poids total de la composition (%) |
|---|---|---|---|
| 1 | 0,25% | - | - |
| 2 | 0,25% | 0.75% | - |
| 3 | 0,25% | 0,75% | 0,02% |
| 4 | 0,5% | 0,5% | 0,02% |
| 5 | 0,25% | 0,25% | 0,01% |
| 6 | 0,1% | 0,1% | 0,01% |
| 7 | 0,01% | 0,01% | 0,01% |
| 8 | 0,75% | 0,25% | 0,02% |
| 9 | 0,5% | - | - |

Nous réalisons également les tests décrits ci-dessus à partir de d'extraits se différenciant par leur solvant d'extraction, pour la graine et la racine d'angélique. En particulier, nous réalisons des tests à partir d'un extrait aqueux, d'un extrait hydroglycolique, d'un extrait hydro-alcoolique, hydro-glycériné, hydro-alcoolique glycériné de graine ou de racine. Les résultats de la présente invention sont confirmés.

### Exemple 2 : Effet d'un extrait hydro-alcoolique de graines d'angélique ou d'un extrait aqueux de racines d'angélique et/ou du mélange des deux pour stimuler la synthèse des protéoglycanes et hydratation en profondeur

Les expériences ont été réalisées avec un extrait hydro-alcoolique glycériné de graines d'Angélique, un extrait aqueux de racines d'Angélique, et le mélange d'extrait de graines et d'extrait de racines.

L'extrait hydro-alcoolique glycériné de graines utilisé était un extrait hydro-alcoolique glycériné des laboratoires M&L obtenu par extraction dans un mélange eau alcool, par exemple, extraction assistée par ultra-sons, selon le procédé suivant : les graines ont été lavées et séchées par le producteur puis ont été mises dans une solution hydro-alcoolique (50/50) à un ratio de 10% à une température de 50°C pendant 30 minutes sous ultrasons sans agitation. Après extraction, la solution a été filtrée à 0,2µm. La solution hydro-alcoolique a été diluée à 100% dans la glycérine.

L'extrait aqueux de racines d'angélique était une eau d'Angélique, par exemple Végébios d'angélique (marque de commerce) de la société Solabia (France) obtenu par extraction aqueuse.Le transcriptome est l'ensemble des ARN messager (molécules servant de matrice pour la synthèse des protéines) issu de l'expression d'une partie du génome d'un tissu cellulaire ou d'un type de cellule. La caractérisation et la quantification du transcriptome dans un tissu donné et dans des conditions données permettent d'identifier les gènes actifs, de déterminer les mécanismes de régulation d'expression des gènes et de définir les réseaux d'expression des gènes. Une des techniques utilisées pour mesurer simultanément le niveau d'expression d'un grand nombre de types différents d'ARN messager est celle de la puce à ADN. Les puces à ADN ont permis de mesurer et de visualiser très rapidement les différences d'expression entre les gènes et ceci à l'échelle d'un génome complet.

### 1. Matériel et méthode

### 1.1 Préparation des échantillons :

Des compositions comprenant indépendamment un extrait hydro-alcoolique glycériné de graines d'Angélique, un extrait aqueux de racines d'Angélique ou le mélange des deux extraits comprenant au moins l'extrait hydro-alcoolique glycériné de graines d'Angélique ont été préparées aux différentes concentrations présentées dans l'exemple 1 et utilisées afin d'effectuer des analyses transcriptomiques. Après avoir réalisé un test de viabilité cellulaire tel que décrit dans l'article de Pongsavee. M *et al* [4] qui a permis de déterminer pour chaque composition la concentration maximale non toxique diluée à appliquer, 5 explants de peau provenant de lifting du visage d'un patient ont été incubés indépendamment en présence de ces compositions pendant 24h dans du DMEM (Dulbecco/Vogt modified Eagle's minimal essential medium) sans sérum à 37°C en atmosphère humide à 5% de CO₂. Un explant de peau non traité a été incubé dans les mêmes conditions. Le tableau 3 récapitule les concentrations appliquées pour chaque composition.

**Tableau 3 : concentrations maximales non toxiques exprimées en % pour l'extrait hydro-alcoolique glycériné de graines d'Angélique ou l'extrait aqueux de racines d'Angélique ou le mélange de l'extrait hydro-alcoolique glycériné de graines et l'extrait aqueux de racines**

| **Composition** | **%** |
|---|---|
| extrait hydro-alcoolique glycériné de graines d'angélique | 0,5 |
| extrait aqueux de racines d'angélique | 0,5 |
| Mélange extrait hydro-alcoolique glycériné de graines et extrait aqueux de racines d'angélique | 0,5+0,5 |

### 1.2 Extraction des ARN totaux et amplification linéaire

Après incubation, les explants de peaux ont été broyés puis les ARN totaux issus de chaque explant ont été extraits en utilisant le RNeasy Kit (Qiagen, Hilden, Germany). Les ARN totaux ont été ensuite quantifiés puis la qualité a été vérifiée en utilisant le BIOanalyzer 2100 avec le RNA 6000 Nano LabChip Kit (Agilent Technologies, Boblingen, Germany). Un RIN entre 7,5 et 10 a attesté de la bonne qualité des ARN totaux issus de chaque explant préalablement incubé ou non avec les différentes compositions précitées

### 1.3 Mesure de l'expression génique sur puce oligonucléotide et acquisition des data :

Les puces oligonucléotides Affymetrix Human Gene 1.0 ST Whole Genome ont été utilisées pour chaque analyse d'expression génique (GeneChip HU Gene 1.0, Affymetrix, Santa Clara, USA).

Le protocole expérimental utilisé est décrit dans la publication de Seitz *et al* [5].

Plus simplement, les cycles de rétrotranscription et d'amplification des ARN totaux en ARNc puis en ADNc ont été réalisés à l'aide des kits Ambion WT Expression (Ambion, Applied Biosystem, USA) et du kit GeneChip WT terminal Labelling and Hybridization User (Affymetrix). L'hybridation des puces et les mesures ont été réalisées par les Microarray Facility Tubingen.

Les Puces ont ensuite été scannées en utilisant le scanner GCS3000 Gene Chip (Affymetrix) et le software GCOS 1.4. Les images scannées ont été ensuite analysées en utilisant la console Expression 1.0 (Affymetrix) afin de convertir des mesures d'intensité lumineuse en données numériques et de normaliser ces données. L'analyse complète des données et une analyse statistique ont été réalisées grâce au logiciel Genespring GX 11 (Agilent Technologies) sur la base, en particulier, d'algorithmes de classification. Des différences d'expression de gènes ainsi obtenues suite à l'application des différentes compositions comprenant indépendamment soit l'extrait hydro-alcoolique glycériné de graines d'Angélique, l'extrait aqueux de racines d'Angélique ou le mélange des deux extraits dont l'extrait hydro-alcoolique glycériné de graines d'Angélique sur des explants de peau par rapport à un explant non traité ont été rassemblées dans le tableau 4 ci-dessous. La valeur de l'expression différentielle de gènes exprimés entre la peau non traitée et la peau traitée avec les différentes compositions précitées présente une p-value < 0,01 (valeur statistique très significative).

**Tableau 4 : différence d'expression génique et signification biologique des gènes suite à l'application des différents extraits d'Angélique et du mélange de deux extraits dont l'extrait hydro-alcoolique glycériné de graines d'Angélique sur explants de peau par rapport à un même explant non traité.**

| GENES | dbEST | Fonction | STATUT: stimulation par rapport à la peau non traitée |
|---|---|---|---|
| FBN1 fibrilline1 | NM_000138.4 | glycoprotéine de la matrice extracellulaire constituant les fibrilles et permettant l'élasticité en limitant les forces de tension dans le derme | X3 dans l'extrait hydro-alcoolique glycériné de graines d'Angélique x3,5 dans le mélange extrait de graines et extrait de racines d'Angélique |
| LOX lysyl oxidase | BC074820.2 | enzyme utilisant le cuivre comme cofacteur initiant la réticulation entre fibres de collagènes et élastines | X3,5 dans le mélange extrait hydro-alcoolique glycériné de graines et d'extrait aqueux de racines d'angélique |
| PLOD1 procollagènelysine, 2-oxoglutarate 5-dioxygénase 1 | NM_000302.3 | enzyme qui joue un rôle crucial dans la stabilité des liaisons intermoléculaires des constituants de la matrice extracellulaire | X3 dans l'extrait hydro-alcoolique glycériné de graines d'angélique X3 dans le mélange extrait hydro-alcoolique glycériné de graines et extrait aqueux de racines d'angélique |
| LUM lumican | NM_002345.3 | protéoglycane appartenant à la famille des kératanes sulfates jouant un rôle dans l'organisation spatiale des fibres de collagènes et fibrillines de par ses charges hydrophiliques | X2,5 dans le mélange extrait hydro-alcoolique glycériné de graines et extrait aqueux de racines d'angélique |
| MMP3 matrix métallopeptidase 3 | NM_002422.3 | enzyme impliquée dans la dégradation de la matrice extracellulaire | Inhibition : -7,5 x dans le mélange extrait hydro-alcoolique glycériné de graines et extrait aqueux de racines d'angélique |
| MMP7 matrix métallopeptidase 7 | NM_002423.3 | enzyme impliquée dans la dégradation de la matrice extracellulaire | Inhibition : -4,5 x dans le mélange extrait hydro-alcoolique glycériné de graines et extrait aqueux de racines d'angélique |

Dans le tableau précité, Xn représente le facteur de stimulation par rapport à la peau non traitée. Dans le tableau 4 ci-dessus, seule la stimulation a été indiquée, l'absence de stimulation en présence de l'extrait hydro-alcoolique glycériné de graines ou aqueux de racines ou du mélange des deux n'a pas été indiquée.

### 1.4 Validation des données d'expression génique par PCR en temps réel

Afin de valider au niveau génique, par une autre méthode, ces données, une expérience de QPCR a été réalisée sur deux gènes cibles LUM, VCAN, MMP3 et LOX impliqués respectivement dans l'hydratation du derme et la stabilité du réseau fibrillaire constituant la matrice extracellulaire (MEC). Pour cela, des amorces en position 3' et 5 'de chaque gène ont été sélectionnées puis synthétisées afin d'amplifier chaque gène pris séparément. Le tableau 5 regroupe la séquence et les caractéristiques de ces amorces.

**Tableau 5 : séquence et caractéristiques des amorces permettant d'amplifier LUM, VCAN, MMP3 et LOX**

| Amorces | Séquences | SEQ ID NO | TM | %GC |
|---|---|---|---|---|
| LUM L | 5'-tggaggtcaatcaacttgagaa-3 | 1 | 59 | 41 |
| LUM R | 5'-caaacgcaaatgcttgatctt-3 | 2 | 60 | 38 |
| VCAN L | 5'-gcacctgtgtgccaggata-3 | 3 | 60 | 58 |
| VCAN R | 5'-cagggattagagtgacattcatca-3 | 4 | 60 | 42 |
| GAPDH L | 5'-agccacatcgctcagacac-3 | 5 | 60 | 58 |
| GAPDH R | 5'-gcccaatacgaccaaatcc-3 | 6 | 60 | 53 |
| MMP3 L | 5'-cagggattagagtgacattcatca-3 | 7 | 59 | 30 |
| MMP3 R | 5'-ttcagctatttgcttgggaaa-3 | 8 | 59 | 38 |
| LOX L | 5'-tgggaatggcacagttgtc-3 | 9 | 59 | 53 |
| LOX R | 5'-aaacttgctttgtggccttc-3 | 10 | 60 | 45 |

Une étape de rétrotranscription a été tout d'abord réalisée en utilisant le système Superscript First-Srand Synthesis System for RT-PCR (Invitrogen Corp, Carlsbad, CA) des ARN issus des explants de peau précédant, le protocole expérimental étant décrit dans la publication de Devilard *et al* [6].

Les expériences de PCR en temps réel ont été réalisées en utilisant le LightCycler 2.0 Detection (Roche Diagnostic, Suisse) en triplicate avec la GAPDH comme gène de référence et contrôle interne. Le kit d'amplification utilisé dans cette expérience est le FastStart DNA Master plus SYBR Green I Master Mix (Roche Diagnostics, Suisse décrit dans la publication de Archambaud et al, STAT6 deletion converts the Th2 inflammatory pathology afflicting Lat(Y136F) mice into a lymphoproliferative disorder involving Th1 and CD8 effector T cells. J Immunol. 2009 Mar 1;182(5):2680-9. [7]).

### 2. RESULATS

### 2.1 Profil d'expression génique (« gene profiling »)

2.1.1. Impact sur l'hydratation du derme de l'extrait de graines d'Angélique, du mélange de l'extrait de graines d'angélique et de l'extrait aqueux de racines d'Angélique.

Les résultats des analyses d'expression de gènes indiqués dans le tableau 4 ont permis de montrer que le gène LUM codant pour le lumican, (Impaired skin wound healing in lumican-null mice. Yeh JT, Yeh LK, Jung SM, Chang TJ, Wu HH, Shiu TF, Liu CY, Kao WW, Chu PH. Br J Dermatol. 2010 Dec;163(6): 1174-80. [11]) protéoglycane essentiel pour l'organisation du réseau de fibres, collagènes et élastines constituant la matrice extracellulaire, voit son expression génique multipliée par 2,5 avec le mélange extrait hydro-alcoolique glycériné de graines et d'extrait aqueux de racines d'Angélique par rapport à la peau non traitée. Aucune expression ne fut retrouvée dans chaque extrait pris séparément. De ce fait, il existe un réel effet synergique du mélange comprenant l'extrait hydro-alcoolique glycériné de graines, l'extrait aqueux de racines d'Angélique qui permettent d'hydrater et de maintenir une bonne hydratation du derme de part la stimulation de l'expression du versican et du lumican, deux protéoglycanes très impliqués dans cette fonction en captant efficacement l'eau grâce à leur charges hydrophyliques et à l'organisation correcte de ce réseau constituant la matrice extracellulaire.
2.1.2. Impact sur le renforcement de la matrice extracellulaire dermique de l'extrait de graines d'Angélique, du mélange de l'extrait de graines d'angélique et de l'extrait aqueux de racines d'Angélique.

### A/ Stimulation des constituants de la matrice extracellulaire

Les résultats des analyses d'expression de gènes regroupés dans le tableau 4 ont permis de montrer que l'expression du gène FBN1, glycoprotéines et protéines constituant la matrice extracellulaire et son organisation structurelle particulière tel que décrit dans Szauter KM et al., « A novel fibrotic disorder associated with increased dermal fibroblast proliferation and downregulation of genes of the microfibrillar network. ». Br J Dermatol. 2010 Nov;163(5):1102-15 [12], a été multipliée par 3 pour la fibrilline 1 dans l'extrait hydro-alcoolique glycériné de graines d'Angélique par rapport à la peau non traitée. Le mélange composé d'extrait de graines et extrait aqueux de racines d'Angélique induit par 3,5 la stimulation de l'expression de la fibrilline 1.

De ce fait, l'extrait hydro-alcoolique glycériné de graines d'Angélique et le mélange constitué d'extrait de graines et d'extrait aqueux de racines d'Angélique renforcent efficacement la matrice extracellulaire, le maintiennent son architecture spécifique à ses fonctions ainsi que le renforcement entre derme et épiderme au niveau de la jonction dermoépidermique. Il existe un réel effet synergique en ce qui concerne le mélange extrait de graines et extrait de racines d'Angélique car la surexpression de la fibrilline 1 est supérieure par rapport aux extraits pris séparément.

### B/Stimulation des enzymes permettant de réticuler les fibres entre elles

Les résultats des analyses d'expression de gènes regroupés dans le tableau 3 ont permis de montrer que l'expression des gènes PLOD1 et LOX a été multipliée par 3 pour PLOD1 dans l'extrait hydro-alcoolique glycériné de graines d'Angélique et dans le mélange extrait de graines et extrait de racines d'angélique et par 3,5 pour LOX dans le mélange extrait hydro-alcoolique glycériné de graines et extrait aqueux de racines d'Angélique par rapport à la peau non traitée. Aucune surexpression de LOX ne fut retrouvée dans chaque extrait pris séparément. De ce fait, on observe ainsi un réel effet synergique du mélange extrait hydro-alcoolique glycériné de graines et de l'extrait aqueux de racines d'Angélique.

Ces deux gènes LOX et PLOD1 codent respectivement pour des enzymes impliquées dans les réactions initiales de réticulation entre les fibres d'élastines, collagènes et fibrillines (Noblesse E, Cenizo V, Bouez C, Borel A, Gleyzal C, Peyrol S, Jacob MP, Sommer P, Damour O. Lysyl oxidase-like and lysyl oxidase are present in the dermis and epidermis of a skin equivalent and in human skin and are associated to elastic fibers. J Invest Dermatol. 2004 Mar;122(3):621-30 [13]) ou dans la stabilisation de ces liaisons (Takaluoma K, Hyry M, Lantto J, Sormunen R, Bank RA, Kivirikko KI, Myllyharju J, Soininen R. Tissue-specific changes in the hydroxylysine content and cross-links of collagens and alterations in fibril morphology in lysyl hydroxylase 1 knock-out mice. J Biol Chem. 2007 Mar 2;282(9):6588-96 [14]).

De ce fait, le mélange extrait hydro-alcoolique glycériné de graines et extrait aqueux de racines d'Angélique ainsi que l'extrait hydro-alcoolique glycériné de graines d'Angélique à travers la stimulation de LOX et PLOD1 permettent de renforcer et maintenir l'architecture de la matrice extracellulaire.

### C/Inhibition de la dégradation de la matrice extracellulaire par inhibition de l'expression des MMPs

Les résultats des analyses d'expression de gènes regroupés dans le tableau 4 ont permis de montrer que l'expression du gène MMP3 est divisée par 7,5 dans le mélange extrait hydro-alcoolique glycériné de graines et extrait aqueux de racines d'Angélique par rapport à la peau non traitée. Aucune inhibition de l'expression ne fut retrouvée dans chaque extrait pris séparément. De ce fait, il existe un réel effet synergique de ce mélange extrait de graines et extrait de racines d'Angélique. Ce gène code pour la metallopeptidase 3 ou stromélysine 1, enzyme impliquée dans la dégradation de la matrice extracellulaire (Lindner D, Zietsch C, Becher PM, Schulze K, Schultheiss HP, Tschöpe C, Westermann D. Differential expression of matrix metalloproteases in human fibroblasts with different origins. Biochem Res Int. 2012;2012:875742 [15]).

De ce fait, le mélange, à travers l'inhibition de l'expression de MMP3, protège la matrice extracellulaire de sa dégradation et ainsi permet le renforcement et le maintient de son architecture. Par ailleurs, le mélange extrait hydro-alcoolique glycériné de graines et extrait aqueux de racines d'Angélique inhibe par 4,5 fois également l'expression du gène *MMP7* codant pour une autre metalloprotéase impliquée dans la dégradation de la matrice extracellulaire. Aucune inhibition de l'expression ne fut retrouvée dans chaque extrait pris séparément.

Cet exemple démontre donc clairement que la composition comprenant un mélange d'extrait de graines et d'extrait de racines d'angélique a un effet synergique sur l'hydratation de la peau au niveau de l'épiderme et du derme.

### 2.2 PCR en temps réel

Les résultats de PCR en temps réel permettant de valider sur les gènes LUM, VCAN, LOX et MMP3 les données de micro-array sont regroupées dans le tableau 6.

**Tableau 6 : expression génique de LUM, VCAN, LOX et MMP3 par RT-QPCR suite à l'application de l'extrait hydro-alcoolique glycériné de graines, extrait aqueux de racines ou du mélange extrait aqueux de racines et extrait de graines d'angélique sur explants de peau par rapport à un même explant non traité.**

| GENES/Statut | Extrait hydro-alcoolique glycériné de graines angélique | Extrait aqueux de racines angélique | Mélange extrait de graines et extrait aqueux de racines |
|---|---|---|---|
| LUM | NS | NS | X3 |
| VCAN | X5 | NS | NS |
| LOX | NS | NS | X2 |
| MMP3 | NS | NS | Inhibition : -3X |

Dans le tableau ci-dessous NS signifie Non Stimulé

L'expression du gène LUM est multipliée par 3 (x3) dans le mélange comprenant l'extrait hydro-alcoolique glycériné de graines et l'extrait aqueux de racines d'Angélique. Aucune surexpression ne fut retrouvée dans les différents extraits pris séparément. En ce qui concerne le gène VCAN, son expression est multipliée par (X5) dans l'extrait hydro-alcoolique glycériné de graines. Aucune surexpression ne fut retrouvée dans l'extrait aqueux de racines ou bien dans le mélange extrait de graines et extrait aqueux de racines d'Angélique. L'expression du gène LOX est multipliée par 2 (x2) dans le mélange comprenant l'extrait hydro-alcoolique glycériné de graines et l'extrait aqueux de racines d'Angélique. Aucune surexpression ne fut retrouvée dans les différents extraits pris séparément. L'expression du gène MMP3 est inhibée par 3 (-x3) dans le mélange comprenant l'extrait hydro-alcoolique glycériné de graines et l'extrait aqueux de racines d'Angélique. Aucune surexpression ne fut retrouvée dans les différents extraits pris séparément.

Les résultats obtenus lors de cette expérience sont identiques à ceux obtenus lors de l'expérience sur puces à ADN ce qui confirme et valide les données de micro-arrays.

Cet exemple démontre clairement par une analyse transcriptomique ex-vivo par Puces à ADN que la composition de l'invention permet de stimuler la synthèse des protéoglycanes, d'empêcher la dégradation de la matrice extracellulaire où ils sont localisés et assurent leur fonctions, de renforcer, de ce fait, l'hydratation en profondeur de la peau.

Nous réalisons également les tests décrits ci-dessus à partir de d'extraits se différenciant par leur solvant d'extraction, pour la graine et la racine d'angélique. En particulier, nous réalisons des tests à partir d'un extrait aqueux, d'un extrait hydroglycolique, d'un extrait hydro-alcoolique, hydro-glycériné, hydro-alcoolique glycériné de graine ou de racine. Les résultats de la présente invention sont confirmés.

### Exemple 3 : Exemple de composition cosmétique pour le soin du cheveu (shampooing) selon la présente invention.

Une composition de base décrite dans le tableau 7 ci-dessous a été préparée par mélange des différents composants indiqués dans le tableau 7 ci-dessous :

**Tableau 7 : composition de base pour les cheveux**

| Ingrédients | Quantité en % poids par rapport au poids total de la composition (%) |
|---|---|
| Sodium laureth sulfate | 10-15 |
| Bétaïne | 1-5 |
| Decyl glucoside | 1-5 |
| Polyquaternium-7 | 0,2-0,5 |
| PEG-7 glyceryl cocoate | 1-4 |
| Solubilisant | 0,5-1 |
| Parfum | Qs |
| Conservateurs, régulateur de pH | Qs |
| Eau | qsp 100% |

La composition de base précitée a été préparée selon le procédé suivant : les tensioactifs ont été introduits à température ambiante, c'est-à-dire entre 20 et 25°C sous agitation à 500 tours par minutes (t/min) l'un après l'autre jusqu'à dissolution respective. Le mélange parfum et solubilisant préalablement préparé a été ajouté. Les conservateurs ont été ensuite ajoutés et le pH ajusté entre 5 et 6,5.

A partir de cette composition de base ont été fabriquées différentes compositions conformes en ajoutant différents extraits en différentes quantités :
- des compositions comprenant de l'huile essentielle de racines d'angélique provenant des Laboratoires Rosier Davenne
- des compositions comprenant de l'huile essentielle de graines d'angélique de la société In Lustrys.

Les pourcentages en poids des différents extraits de racines et graines d'angélique sont indiqués dans le tableau 8 suivant.

**Tableau 8: pourcentage en poids des extraits par rapport au poids de la composition**

| Compositions | % en poids d'huile essentielle de graines d'angélique par rapport au poids total de la composition (%) | % en poids d'huile essentielle de racines d'angélique par rapport au poids total de la composition (%) |
|---|---|---|
| N°1 | 0,004 | 0,006 |
| N°2 | 0,006 | 0,004 |
| N°3 | 0,01 | - |
| N°4 | - | 0,01 |
| N°5 | 0,002 | 0,004 |
| N°6 | 0,004 | 0,002 |
| N°7 | 0,1 | - |
| N°8 | - | 0,1 |

### Exemple 4 : Exemple de composition cosmétique pour le soin du cheveu (après-shampooing) selon la présente invention.

Une composition de base décrite dans le tableau 9 ci-dessous a été préparée par mélange des différents composants indiqués dans le tableau 9 ci-dessous :

**Tableau 9 : Composition de base pour les cheveux**

| Ingrédients | Quantité en % poids par rapport au poids total de la composition (%) |
|---|---|
| Distearoyléthyl dimonium chlorure et cetearyl alcool (auto-émulsionnant) | 9-12 |
| Glycérine | 1-5 |
| Huile d'amande | 1-2 |
| Silicones | 2-7 |
| Polyquaternium-10 | 0,1-0,3 |
| Parfum | Qs |
| Conservateurs | Qs |
| Eau | qsp 100% |

La composition de base a été préparée selon le procédé suivant : l'eau et la glycérine ont été chauffés à 80°C sous agitation 1500 t/min, le polyquaternium a été solubilisé sous agitation 1500 t/min, l'auto-émulsionnant a été ajouté et agité à 1500 t/min jusqu'à dissolution (au minimum 30 minutes). L'huile végétale et le silicone non volatile ont été ensuite ajoutés sous agitation à 1500 t/min. La composition a été émulsionnée à 2000 t/min pendant 5 minutes, puis refroidie à 40°C. Après refroidissement, les autres constituants ont été ajoutés.

A partir de cette composition de base ont été fabriquées différentes compositions en ajoutant différents extraits en différentes quantités :
- des compositions comprenant de l'huile essentielle de racines d'angélique provenant des Laboratoires Rosier Davenne
- des compositions comprenant de l'huile essentielle de graines d'angélique de la société In Lustrys.

Les pourcentages en poids des différents extraits de racines et graines d'angélique sont indiqués dans le tableau 10 suivant.

**Tableau 10: pourcentage en poids des extraits par rapport au poids de la composition**

| Compositions | % en poids d'huile essentielle de graines d'angélique par rapport au poids total de la composition (%) | % en poids d'huile essentielle de racines d'angélique par rapport au poids total de la composition (%) |
|---|---|---|
| N°1 | 0,004 | 0,006 |
| N°2 | 0,006 | 0,004 |
| N°3 | 0,01 | - |
| N°4 | - | 0,01 |
| N°5 | 0,002 | 0,004 |
| N°6 | 0,004 | 0,002 |
| N°7 | 0,1 | - |
| N°8 | - | 0,1 |

### Exemple 5 : Exemple de composition cosmétique pour le soin du cheveu (masque) selon la présente invention.

Une composition de base décrite dans le tableau 11 ci-dessous a été préparée par mélange des différents composants indiqués dans le tableau 11 ci-dessous :

**Tableau 11 : composition de base pour les cheveux**

| Ingrédients | Quantité en % poids par rapport au poids total de la composition (%) |
|---|---|
| Distearoyléthyl dimonium chlorure et cétéaryl alcool (auto-émulsionnant) | 3-7 |
| Alcool cétylique | 1-2 |
| Glycérine | 1-5 |
| Huile d'amande | 1-2 |
| Silicone | 3-7 |
| Polyquaternium | 0,1-1 |
| Parfum | Qs |
| Conservateurs | Qs |
| Eau | qsp 100% |

La composition de base a été préparée selon le procédé suivant : l'eau et la glycérine ont été chauffés à 80°C sous agitation 1500 t/min, le polyquaternium a été solubilisé sous agitation 1500 t/min, puis l'auto-émulsionnant a été ajouté et agité à 1500 t/min jusqu'à dissolution pendant au minimum 30 minutes. L'huile végétale, l'alcool cétylique et la silicone non volatile ont été ajoutés sous agitation. L'ensemble a été ensuite émulsionner à 2000 t/min pendant 5 minutes, refroidit à 40°C. Les autres constituants ont été alors ajoutés dans le mélange en-dessous de 40°C.

A partir de cette composition de base ont été fabriquées différentes compositions conformes à la présente invention en ajoutant différents extraits en différentes quantités :
- des compositions comprenant de l'huile essentielle de racines d'angélique provenant des Laboratoires Rosier Davenne
- des compositions comprenant de l'huile essentielle de graines d'angélique de la société In Lustrys.

Les pourcentages en poids des différents extraits de racines et graines d'angélique sont indiqués dans le tableau 12 suivant.

**Tableau 12: pourcentage en poids des extraits par rapport au poids de la composition**

| Compositions | % en poids d'huile essentielle de graines d'angélique par rapport au poids total de la composition (%) | % en poids d'huile essentielle de racines d'angélique par rapport au poids total de la composition (%) |
|---|---|---|
| N°1 | 0,004 | 0,006 |
| N°2 | 0,006 | 0,004 |
| N°3 | 0,01 | - |
| N°4 | - | 0,01 |
| N°5 | 0,002 | 0,004 |
| N°6 | 0,004 | 0,002 |
| N°7 | 0,1 | - |
| N°8 | - | 0,1 |

### Exemple 6 : Effet d'une huile essentielle de graines d'Angélique ou d'une huile essentielle de racines d'Angélique et/ou du mélange des deux pour renforcer et reconstituer les fibres capillaires des cheveux et stimuler la pousse et la microcirculation au niveau du cuir chevelu.

Le transcriptome est l'ensemble des ARN messager (molécules servant de matrice pour la synthèse des protéines) issu de l'expression d'une partie du génome d'un tissu cellulaire ou d'un type de cellule. La caractérisation et la quantification du transcriptome dans un tissu donné et dans des conditions données permettent d'identifier les gènes actifs, de déterminer les mécanismes de régulation d'expression des gènes et de définir les réseaux d'expression des gènes. Une des techniques utilisées pour mesurer simultanément le niveau d'expression d'un grand nombre de types différents d'ARN messager est celle de la puce à ADN. Les puces à ADN ont permis de mesurer et de visualiser très rapidement les différences d'expression entre les gènes et ceci à l'échelle d'un génome complet.

### 1. Matériel et méthode

### 1.1 Préparation des échantillons :

Des compositions comprenant indépendamment une huile essentielle de racines d'Angélique, une huile essentielle de graines d'Angélique ou le mélange de deux huiles essentielles comprenant au moins l'huile essentielle de graines d'Angélique ont été préparées aux différentes concentrations présentées dans les exemples 3, 4 et 5 et utilisées afin d'effectuer des analyses transcriptomiques. Après avoir réalisé un test de viabilité cellulaire tel que décrit dans l'article de Pongsavee.M *et al* [4] qui a permis de déterminer pour chaque composition la concentration maximale non toxique diluée à appliquer,5 explants de peau provenant de lifting du visage d'une patiente ont été incubés indépendamment en présence de ces compositions pendant 24h dans du DMEM (Dulbecco/Vogt modified Eagle's minimal essential medium) sans sérum à 37°C en atmosphère humide à 5% de CO₂. Un explant de peau non traité a été incubé dans les mêmes conditions. Le tableau 13 récapitule les concentrations appliquées pour chaque composition.

**Tableau 13 : concentrations maximales non toxiques exprimées en % pour l'huile essentielle de racines d'Angélique, pour l'huile essentielle de graines d'Angélique ou le mélange des deux huiles essentielles**

| **Composition** | **%** |
|---|---|
| huile essentielle de graines d'angélique | 0,00001 |
| huile essentielle de racines d'angélique | 0,00001 |
| huile essentielle de racines d'angélique et huile essentielle de graines d'angélique | 0,00001+0,00001 |

### 1.2 Extraction des ARN totaux et amplification linéaire

Après incubation, les explants de peaux ont été broyés puis les ARN totaux issus de chaque explant ont été extraits en utilisant le RNeasy Kit (Qiagen, Hilden, Germany). Les ARN totaux ont été ensuite quantifiés puis la qualité a été vérifiée en utilisant le BlOanalyzer 2100 avec le RNA 6000 Nano LabChip Kit (Agilent Technologies, Boblingen, Germany). Un RIN entre 7,5 et 10 a attesté de la bonne qualité des ARN totaux issus de chaque explant préalablement incubé ou non avec les différentes compositions précitées.

### 1.3 Mesure de l'expression génique sur puce oligonucléotide et acquisition des data :

Les puces oligonucléotides Affymetrix Human Gene 1.0 ST Whole Genome ont été utilisées pour chaque analyse d'expression génique (GeneChip HU Gene 1.0, Affymetrix, Santa Clara, USA).

Le protocole expérimental utilisé est décrit dans la publication de Seitz *et al* [5]. Plus simplement, les cycles de rétrotranscription et d'amplification des ARN totaux en ARNc puis en ADNc ont été réalisés à l'aide des kits Ambion WT Expression (Ambion, Applied Biosystem, USA) et du kit GeneChip WT terminal Labelling and Hybridization User (Affymetrix). L'hybridation des puces et les mesures ont été réalisées par les Microarray Facility Tubingen.

Les Puces ont ensuite été scannées en utilisant le scanner GCS3000 Gene Chip (Affymetrix) et le software GCOS 1.4. Les images scannées ont été ensuite analysées en utilisant la console Expression 1.0 (Affymetrix) afin de convertir des mesures d'intensité lumineuse en données numériques et de normaliser ces données. L'analyse complète des données et une analyse statistique ont été réalisées grâce au logiciel Genespring GX 11 (Agilent Technologies) sur la base, en particulier, d'algorithmes de classification. Des différences d'expression de gènes ainsi obtenues suite à l'application des différentes compositions comprenant indépendamment soit l'huile essentielle de racines d'Angélique, soit l'huile essentielle de graines d'Angélique ou le mélange de deux huiles essentielles sur des explants de peau par rapport à un explant non traité ont été rassemblées dans le tableau 14 ci-dessous. La valeur de l'expression différentielle de gènes exprimés entre la peau non traitée et la peau traitée avec les différentes compositions précitées présente une p-value < 0,01 (valeur statistique très significative).

**Tableau 14 : différence d'expression génique et signification biologique des gènes suite à l'application de l'huile essentielle de racines d'Angélique ou de l'huile essentielle de graines d'Angélique et du mélange des deux huiles essentielles sur explants de peau par rapport à un même explant non traité.**

| GENES | DbEST | Fonction | STATUT: |
|---|---|---|---|
| | | | stimulation par rapport à la peau non traitée |
| KRT34 kératine 34 | NM_021013.3 | Protéine de type I qui s'hétérodimérise avec les protéines de type II pour former les cheveux et les ongles | X2,5 dans le mélange HE racines et graines angélique |
| KRTAP5-4 protéine associée à la kératine 5-4 | NM_001012709.1 | Protéine formant une matrice autour des filaments de kératine, renforçant la gaine capillaire | X3 dans l'huile essentielle de graines d'angélique/ x3 dans l'huile essentielle de racines d'angélique/ X4,5 dans le mélange HE racines et graines angélique |
| KRTAP10-7 protéine associée à la kératine 10-7 | NM_198689.2 | Protéine formant une matrice autour des filaments de kératine, renforçant la gaine capillaire | X2 dans l'huile essentielle de graines d'angélique/ x2 dans l'huile essentielle de racines d'angélique/ X3,5 dans le mélange HE racines et graines angélique |
| ANGPTL4 angiopoiétine 4 | AB056477.1 | Protéine induite par l'hypoxie et sécrétée par les cellules endothéliales permettant de stimuler l'angiogenèse et la pousse des cheveux | X3,5 dans le mélange HE racines et graines angélique |
| RPTN Répétine | NM_001122965.1 | Cette protéine joue un rôle dans la résistance mécanique des fibres capillaires | X7,5 dans le mélange HE racines et graines angélique |

Dans le tableau précité, Xn représente le facteur de stimulation par rapport à la peau non traitée, HE signifie Huile Essentielle. Dans le tableau 14 ci-dessus, seule la stimulation a été indiquée, l'absence de stimulation en présence des huiles essentielles ou de leur mélange n'a pas été indiquée.

### 1.4 Validation des données d'expression génique par PCR en temps réel

Afin de valider au niveau génique, par une autre méthode, ces données, une expérience de QPCR a été réalisée sur deux gènes cibles *KRT34,* et *RPTN* impliqués respectivement dans la kératinisation et la résistance des fibres capillaires. Pour cela, des amorces en position 3' et 5 'de chaque gène ont été sélectionnées puis synthétisées afin d'amplifier chaque gène pris séparément. Le tableau 15 regroupe la séquence et les caractéristiques de ces amorces.

**Tableau 15 : séquence et caractéristiques des amorces permettant d'amplifier KRT34 et RPTN**

| Amorces | Séquences | SEQ ID NO | TM | %GC |
|---|---|---|---|---|
| KRT34 L | 5'-ggagagtgaggactgcaagc-3 | 11 | 60 | 60 |
| KRT34 R | 5'-ccacaggagttgccactagc-3 | 12 | 60 | 60 |
| RPTN L | 5'-gctcttggctgagtttggag-3 | 13 | 60 | 55 |
| RPTN R | 5'-aggttcaagatggtttccaca -3 | 14 | 59 | 43 |
| GAPDH L | 5'-agccacatcgctcagacac-3 | 5 | 60 | 58 |
| GAPDH R | 5'-gcccaatacgaccaaatcc-3 | 6 | 60 | 53 |

Une étape de rétrotranscription a été tout d'abord réalisée en utilisant le système Superscript First-Srand Synthesis System for RT-PCR (Invitrogen Corp, Carlsbad, CA) des ARN issus des explants de peau précédant, le protocole expérimental étant décrit dans la publication de Devilard *et al* [6].

Les expériences de PCR en temps réel ont été réalisées en utilisant le LightCycler 2.0 Detection (Roche Diagnostic, Suisse) en triplicate avec la GAPDH comme gène de référence et contrôle interne. Le kit d'amplification utilisé dans cette expérience est le FastStart DNA Master plus SYBR Green I Master Mix (Roche Diagnostics, Suisse décrit dans la publication de Archambaud *et al [7].*

### 2. RESULATS

### 2.1 Profil d'expression génique (« gene profiling »)

**2.1.1.** Impact sur la constitution des fibres capillaires et leur renforcement par de l'huile essentielle de graines d'angélique, de l'huile essentielle de racines d'angélique ou un mélange d'huile essentielle de graines d'angélique et d'huile essentielle de racines d'Angélique sur la peau et le cuir chevelu.

Les résultats des analyses d'expression de gènes indiqués dans le tableau 14 ont permis de montrer que l'expression du gène KRT34, codant pour une kératine de type I impliquée dans la formation de la gaine capillaire (New consensus nomenclature for mammalian keratins. Schweizer J, Bowden PE et al. [16]) est multipliée par 2,5 dans le mélange huile essentielle de graines d'Angélique et huile essentielle de racines d'Angélique. Aucune surexpression de ce gène n'a été détectée dans la peau non traitée ainsi que dans la peau traitée respectivement avec indépendamment soit l'huile essentielle de graines d'Angélique, soit l'huile essentielle de racines d'Angélique. Il existe, de ce fait, un réel effet synergique du mélange comprenant l'huile essentielle de racines + l'huile essentielle de graines d'Angélique. Par ailleurs, l'expression de deux autres gènes *KRT75 et KRT31* codant également pour des kératines impliquées dans la constitution des fibres capillaires [16], est respectivement multipliée par 3,5 et par 3 avec l'huile essentielle de graines d'Angélique puis avec l'huile essentielle de racines d'Angélique. Aucune surexpression de *KRT75* ne fut retrouvée dans la peau non traitée ainsi que dans la peau traitée avec le mélange comprenant les deux huiles essentielles racines et de graines d'Angélique. En ce qui concerne KRT31, aucune surexpression ne fut retrouvée dans la peau non traitée, l'expression est multipliée par 2,5 dans le mélange huile essentielle de racines d'Angélique et huile essentielle de graines d'Angélique.

De plus, l'huile essentielle de racines d'angélique, l'huile essentielle de graines d'Angélique et le mélange des deux huiles essentielles stimulent avantageusement l'expression de deux gènes *KRTAP10-7, KRTAP5-4* codant pour des protéines associées aux kératines (Molecular evolution of the keratin associated protein gene family inmammals, role in the evolution of mammalian hair. Dong-Dong Wu, David M Irwin et al [17]). En effet, l'expression de *KRTAP10-7* est multipliée par 2 avec l'huile essentielle de racines d'Angélique, avec l'huile essentielle de graines d'Angélique et par 3,5 avec le mélange des deux huiles traitée. Il existe, de ce fait, un réel effet synergique du mélange comprenant l'huile essentielle de racines et l'huile essentielle de graines d'Angélique. En ce qui concerne *KRTAPS-4,* l'huile essentielle de graines d'Angélique ou l'huile essentielle de racines d'angélique stimulent par 3 son expression. Le mélange de ces deux huiles essentielles d'angélique stimule par 4,5 l'expression de. Aucune surexpression de *KRTAP5-4* ne fut retrouvée dans la peau non traitée. Il existe, de ce fait, un réel effet synergique du mélange comprenant l'huile essentielle de racines et l'huile essentielle de graines d'Angélique.

Le mélange huile essentielle de graines d'Angélique et huile essentielle de racines d'Angélique stimule par 7,5 également l'expression du gène *RPTN* codant pour la répétine (Huber M, Siegenthaler G, Mirancea N, Marenholz I, Nizetic D, Breitkreutz D, Mischke D, Hohl D. Isolation and characterization of human repetin, a member of the fused gene family of the epidermal differentiation complex [18]) protéine assurant la résistance mécanique (« anti-casse ») des fibres capillaires. Aucune surexpression de ce gène n'a été détectée dans la peau non traitée ainsi que dans la peau traitée respectivement avec indépendamment soit l'huile essentielle de graines d'angélique, soit l'huile essentielle de racines d'angélique. Il existe, de ce fait, un réel effet synergique du mélange comprenant l'huile essentielle de racines et l'huile essentielle de graines d'Angélique.

De ce fait, cet exemple démontre clairement que l'huile essentielle de racines d'angélique, l'huile essentielle de graines d'Angélique et le mélange des deux permettent de constituer, de renforcer la gaine des fibres capillaires, d'assurer une résistance mécanique de ses fibres entrainant un effet anti-casse en stimulant efficacement les kératines et protéines associées aux kératines et la répétine.
2.1.2. Impact sur la pousse des cheveux de l'huile essentielle de graines d'angélique, de l'huile essentielle de racines d'angélique et du mélange de l'huile essentielle de graines d'angélique et de l'huile essentielle de racines d'angélique.

L'huile essentielle de graines d'angélique ou l'huile essentielle de racines d'angélique stimulent l'expression du gène *ELK3,* codant pour un facteur de transcription de la famille ETS et entrainant la prolifération cellulaire et ainsi la pousse des cheveux (Nakade K, Zheng H, Ganguli G, Buchwalter G, Gross C, Wasylyk B. The tumor suppressor p53 inhibits Net (ELK3), an effector of Ras/extracellular signal-regulated kinase signaling [19]). Aucune surexpression de ce gène n'a été détectée dans la peau non traitée ainsi que dans la peau traitée avec le mélange des deux huiles essentielles.

De ce fait, l'huile essentielle de racines d'angélique, l'huile essentielle de graines d'Angélique permettent de stimuler la pousse des cheveux.
2.1.3. Impact sur la microcirculation au niveau du cuir chevelu et sur la pousse des cheveux de l'huile essentielle de graines d'angélique, de l'huile essentielle de racines d'angélique et du mélange de l'huile essentielle de graines d'angélique et de l'huile essentielle de racines d'angélique.

L'huile essentielle de graines d'angélique ou l'huile essentielle de racines d'angélique stimulent l'expression du gène *FBLNS,* codant pour une protéine impliquée dans la vasculogénèse stimulant la pousse des cheveux en hypoxie (Guadall A, Orriols M, Rodriguez-Calvo R, Calvayrac O, Crespo J, Aledo R, Martinez-Gonzàlez J, Rodriguez C. Fibulin-5 is up-regulated by hypoxia in endothelial cells through a hypoxia-inducible factor-1 (HIF-1α)-dependent mechanism [20], Preis M, Cohen T, Sarnatzki Y, Ben Yosef Y, Schneiderman J, Gluzman Z, Koren B, Lewis BS, Shaul Y, Flugelman MY. Effects of fibulin-5 on attachment, adhesion, and proliferation of primary human endothelial cells [21]). Aucune surexpression de ce gène n'a été détectée dans la peau non traitée ainsi que dans la peau traitée avec le mélange des deux huiles essentielles. De plus, le mélange huile essentielle de racines et huile essentielle de graines d'angélique stimule par 3.5 l'expression de *ANGPTL4* codant pour une protéine impliquée dans l'angiogenèse en condition hypoxique et stimulant ainsi la pousse des cheveux (Perdiguero EG, Galaup A, Durand M, Teillon J, Philippe J, Valenzuela DM, Murphy AJ, Yancopoulos GD, Thurston G, Germain S. Alteration of developmental and pathological retinal angiogenesis in angptl4-deficient mice [22]). Aucune surexpression de ce gène n'a été détectée dans la peau non traitée ainsi que dans la peau traitée respectivement avec indépendamment soit l'huile essentielle de graines d'Angélique, soit l'huile essentielle de racines d'Angélique. Il existe, de ce fait, un réel effet synergique du mélange comprenant l'huile essentielle de racines et l'huile essentielle de graines d'Angélique.

De ce fait, l'huile essentielle de racines d'angélique, l'huile essentielle de graines d'Angélique et le mélange des deux permettent d'activer la microcirculation au niveau du cuir chevelu et de stimuler en condition hypoxique la pousse des cheveux.

### 2.2 PCR en temps réel

Les résultats de PCR en temps réel permettant de valider sur les gènes KRT34 et RPTN les données de micro-array sont regroupées dans le tableau 16.

**Tableau 16 : expression génique de KRT34 et RPTN par RT-QPCR suite à l'application de l'huile essentielle de racines d'angélique, de l'huile essentielle de graines d'angélique ou du mélange de ces deux huiles essentielles sur explants de peau et cuir chevelu par rapport à un même explant non traité.**

| STATUT /GENES | huile essentielle de racines d'angélique | huile essentielle de graines d'angélique | mélange huile essentielle de racines et de graines d'angélique |
|---|---|---|---|
| KRT 34 | NS | NS | x2,5 |
| RPTN | NS | NS | x7,5 |

| | | | |
|---|---|---|---|
| Dans le tableau ci-dessous, NS signifie non stimulé. | | | |

L'expression du gène *KRT34* est multipliée par 2,5 (x2,5) dans le mélange comprenant l'huile essentielle de racines et l'huile essentielle de graines d'Angélique. Aucune surexpression ne fut retrouvée dans chaque huile essentielle prise séparément. En ce qui concerne le gène *RPTN,* son expression est multipliée par 7,5 (X7,5) dans le mélange comprenant l'huile essentielle de racines et l'huile essentielle de graines d'angélique. Aucune surexpression ne fut retrouvée dans chaque huile essentielle prise séparément.

Les résultats obtenus lors de cette expérience sont identiques à ceux obtenus lors de l'expérience sur puces à ADN ce qui confirme et valide les données de micro-arrays.

Cet exemple démontre clairement par une analyse transcriptomique ex-vivo par Puces à ADN que la composition de l'invention permet de stimuler la synthèse des kératines et des protéines associées aux kératines entrant dans la constitution de la gaine capillaire, de stimuler la microcirculation au niveau du cuir chevelu et d'augmenter la résistance mécanique des fibres capillaires.

### Exemple 7 : Tests d'usages et d'efficacité

Afin de valider l'efficacité et l'action réparatrice au niveau de la fibre capillaire de la composition de l'invention, 38 femmes âgées entre 20 et 55 ans ont été recrutées pour cette étude afin de tester l'activité réparatrice, de la composition ainsi que les qualités « cosmétiques » de cette composition. De ce fait, la composition a été appliquée de deux à sept fois par semaine pendant 28 jours dans les conditions normales d'utilisation.

La composition utilisée est celle décrite dans l'exemple 3 identifiée composition N°1 dans le tableau 8 ci-dessous.

Les caractéristiques des cheveux des femmes ayant participées au test sont résumés dans les tableaux 17 à 19 ci-dessous :

**Tableau 17 : qualité des cheveux en fonction des participants**

| Cheveux | Effectifs | Pourcentage |
|---|---|---|
| Très secs | 1 | 3% |
| Secs | 14 | 37% |
| Normaux | 7 | 18% |
| Mixtes (racines grasses, pointes sèches) | 16 | 42% |
| Total | 38 | 100 % |

**Tableau 18 : longueur des cheveux en fonction des participants**

| Cheveux | Effectifs | Pourcentage |
|---|---|---|
| Courts | 9 | 24% |
| Mi-longs | 21 | 55% |
| Longs | 8 | 21% |
| Total | 38 | 100% |

**Tableau 19 : état des cheveux en fonction des participants**

| Cheveux | Effectifs | Pourcentage |
|---|---|---|
| Abîmés | 15 | 39% |
| Légèrement abîmés | 21 | 55% |
| En bon état | 2 | 5% |
| Total | 38 | 100% |

Le tableau 20 ci-dessous comprend les résultats obtenus et démontre l'efificacité de la composition utilisée.

| Propriétés | Favorable |
|---|---|
| A l'application | |
| Le shampooing lave en douceur: | 97% |
| Ce shampooing s'applique bien sur mes cheveux : | 95% |
| La texture du shampooing est agréable : | 95% |
| La texture du shampooing est fondante : | 95% |
| La texture du shampooing me plaît: | 95% |
| Ce shampooing se rince facilement : | 95% |
| Ce shampooing ne pique pas les yeux : | 95% |
| Le packaging est pratique à utiliser : | 92% |
| Ce shampooing mousse bien : | 92% |
| Le parfum du shampooing est naturel : | 92% |
| Ce shampooing enveloppe les cheveux de douceur: | 89% |
| Ce shampooing procure un sentiment de bien-être : | 87% |
| Le parfum du shampooing est bien dosé : | 87% |
| Ce shampooing laisse les cheveux faciles à coiffer : | 87% |
| Le parfum du shampooing me plaît: | 79% |
| On reconnaît bien le parfum des huiles essentielles : | 79% |

| Après l'application | |
|---|---|
| Mes cheveux sont propres : | 100% |
| Mes cheveux sont brillants : | 100% |
| Mes cheveux sont souples : | 100% |
| Ce shampooing aide à nourrir mes cheveux : | 95% |
| Mes cheveux sont légers : | 95% |
| Mes cheveux sont plus doux : | 95% |
| Mes cheveux sont gainés : | 95% |
| Mes cheveux offrent un toucher incroyablement doux : | 95% |
| Ce shampooing aide à restaurer la fibre de mes cheveux : | 95% |
| Mes cheveux sont protégés : | 95% |
| Mes cheveux sont hydratés : | 92% |
| Mes cheveux sont soyeux : | 92% |
| Mes cheveux ont retrouvé leur douceur : | 92% |
| Mes cheveux sont réparés : | 92% |
| Ce shampooing aide à réparer la fibre de mes cheveux : | 92% |
| Mes cheveux sont fortifiés : | 92% |
| Mes cheveux sont moins cassants : | 92% |
| Ce shampooing aide à combler les brèches de mes cheveux : | 92% |
| Mes cheveux sont moins fragiles : | 92% |
| Ce shampooing est bien adapté aux cheveux secs, abîmés, fragilisés : | 92% |
| Mes cheveux sont plus forts : | 89% |
| Mes cheveux sont plus résistants : | 89% |
| Mes cheveux sont pleins de vie : | 89% |
| Mes cheveux sont renforcés : | 89% |
| Mes cheveux résistent à la casse : | 89% |
| Mes cheveux résistent mieux au brossage : | 89% |
| Mes cheveux semblent restructurés : | 89% |
| Mes cheveux sont protégés de la casse : | 89% |
| J'ai moins de cheveux cassés au brossage : | 89% |
| Le produit a une efficacité anti-casse | 89% |
| Le shampooing laisse un fini agréable sur le cheveu : | 87% |
| Mes cheveux sont moins secs : | 87% |
| Ce shampooing aide à lisser les écailles de mes cheveux : | 87% |
| Le produit aide à sceller les écailles de mes cheveux : | 87% |
| C'est comme si on avait insufflé une nouvelle force à mes cheveux : | 87% |
| Mes cheveux sont transformés : | 87% |
| Mes cheveux sont protégés des agressions extérieures (sèche-cheveux, froid, soleil, brossage...) : | 87% |
| Mes cheveux ont l'air éclatant de santé : | 87% |
| C'est comme si mes cheveux avaient retrouvé de la force | 87% |
| Mes cheveux sont réparés jusqu'au bout des pointes : | 87% |
| Mes cheveux ne sont pas alourdis : | 87% |
| Mes cheveux restent parfumés : | 84% |
| Mes cheveux ont retrouvé une facilité de coiffage : | 84% |
| Mes fourches semblent réparées : | 84% |
| Mes cheveux sont magnifiés : | 82% |
| J'ai moins de fourches : | 82% |
| Je n'ai pas d'électricité statique dans les cheveux : | 82% |

Tel que démontré dans cet exemple, une composition selon l'invention, ici un shampoing permet avantageusement de protéger et renforcer les cheveux tout en traitant et réparant la fibre capillaire.

Un second test d'usage a été réalisé avec la composition cosmétique selon l'invention décrite dans l'exemple 4 ci-dessus identifiée N°1 dans le tableau 10.

Pour ce faire, 38 femmes âgées entre 19 et 54 ans ont été recrutées pour cette étude afin de tester l'activité réparatrice, de la composition ainsi que les qualités « cosmétiques » de cette composition. De ce fait, la composition a été appliquée de une à trois fois par semaine pendant 28 jours dans les conditions normales d'utilisation.

Les caractéristiques des cheveux des femmes ayant participées au test sont résumés dans les tableaux 21 à 24 ci-dessous :

**Tableau 21 : qualité des cheveux en fonction des participants**

| Cheveux | Effectifs | pourcentage |
|---|---|---|
| Très secs | 5 | 13% |
| Secs | 11 | 29% |
| Normaux | 5 | 13% |
| Mixtes (racines grasses, pointes sèches) | 17 | 45% |
| Total | 38 | 100 % |

**Tableau 22 : longueur des cheveux en fonction des participants**

| Cheveux | Effectifs | pourcentage |
|---|---|---|
| Courts | 5 | 13% |
| Mi-longs | 21 | 55% |
| Longs | 12 | 32% |
| Total | 38 | 100% |

**Tableau 23 : coloration ou non des cheveux en fonction des participants**

| Cheveux | Effectifs | pourcentage |
|---|---|---|
| Colorés | 15 | 39% |
| Méchés | 12 | 32% |
| Naturels | 11 | 29% |
| Total | 38 | 100% |

**Tableau 24 : état des cheveux en fonction des participants**

| Cheveux | Effectifs | pourcentage |
|---|---|---|
| Très abîmés | 3 | 8% |
| Abîmés | 25 | 66% |
| Légèrement abîmés | 9 | 24% |
| Pas abimé | 1 | 3% |
| Total | 38 | 100% |

Le tableau 25 ci-dessous comprend les résultats obtenus et démontre l'efificacité de la composition utilisée.

| Propriétés | Favorable |
|---|---|
| A l'application | |
| Cet après-shampooing se rince facilement : | 100% |
| Cet après-shampooing respecte les cheveux et le cuir chevelu : | 97% |
| Cet après-shampooing s'applique bien sur les cheveux : | 95% |
| La texture de cet après-shampooing est agréable : | 95% |
| La texture de cet après-shampooing est fondante : | 95% |
| Cet après-shampooing démêle bien les cheveux : | 92% |
| Cet après-shampooing démêle en douceur les cheveux : | 92% |
| Cet après-shampooing procure un sentiment de bien-être : | 89% |
| Le packaging de cet après-shampooing est pratique à utiliser : | 82% |
| On reconnaît bien le parfum des huiles essentielles : | 82% |
| Le parfum de cet après-shampooing est naturel : | 66% |
| Le parfum de cet après-shampooing est agréable : | 63% |
| Le parfum de cet après-shampooing est bien dosé : | 58% |

| Après chaque utilisation | |
|---|---|
| Cet après-shampooing ne laisse pas d'effet gras sur les cheveux : | 92% |
| Mes cheveux sont parfaitement démêlés : | 92% |
| Mes cheveux sont souples : | 92% |
| Mes cheveux ne sont pas alourdis : | 92% |
| Mes cheveux sont légers : | 89% |
| Mes cheveux sont soyeux : | 89% |
| Cet après-shampooing aide à lisser les écailles de mes cheveux : | 89% |
| Mes cheveux sont faciles à coiffer : | 87% |
| Mes cheveux sont instantanément démêlés : | 87% |
| Cet après-shampooing aide à nourrir mes cheveux : | 87% |
| Mes cheveux ont retrouvé une facilité de coiffage : | 87% |
| Je n'ai pas d'électricité statique dans les cheveux : | 87% |
| Mes cheveux restent parfumés : | 84% |
| Cet après-shampooing laisse un fini agréable sur le cheveu : | 84% |
| Mes cheveux sont hydratés : | 84% |
| Mes cheveux sont brillants : | 84% |
| Mes cheveux sont plus doux : | 84% |
| Mes cheveux sont gainés : | 84% |
| Mes cheveux sont plus forts : | 84% |
| Mes cheveux sont pleins de vie : | 84% |
| Mes cheveux ont retrouvé leur douceur : | 84% |
| Mes cheveux résistent mieux au brossage : | 84% |
| Mes cheveux sont moins cassants : | 84% |
| Mes cheveux sont moins secs : | 82% |
| Mes cheveux sont plus résistants : | 82% |
| Mes cheveux sont réparés : | 82% |
| C'est comme si on avait insufflé une nouvelle force à mes cheveux : | 82% |
| Mes cheveux résistent à la casse : | 82% |
| Mes cheveux sont protégés de la casse : | 82% |
| Mes cheveux sont renforcés : | 82% |
| Cet après-shampooing est bien adapté aux cheveux secs, abîmés, fragilisés : | 82% |
| Mes cheveux sont forts : | 79% |
| Cet après-shampooing aide à restaurer la fibre de mes cheveux : | 79% |
| Cet après-shampooing aide à réparer la fibre de mes cheveux : | 79% |
| Mes cheveux sont transformés : | 79% |
| Mes cheveux semblent restructurés : | 79% |
| Mes cheveux sont protégés des agressions extérieures (sèche-cheveux, froid, soleil, brossage...): | 79% |
| Cet après-shampooing a une efficacité anti-casse : | 79% |
| J'ai moins de fourches : | 79% |
| Mes cheveux sont fortifiés : | 79% |
| C'est comme si mes cheveux avaient retrouvé de la force : | 79% |
| Mes cheveux offrent un toucher incroyablement doux : | 76% |
| Mes cheveux sont magnifiés : | 76% |
| Mes cheveux sont moins fragiles : | 76% |
| J'ai moins de cheveux cassés au brossage : | 76% |
| Mes cheveux sont réparés jusqu'au bout des pointes : | 76% |
| Cet après-shampooing aide à combler les brèches de mes cheveux : | 74% |

Tel que démontré dans cet exemple, une composition selon l'invention, ici un après shampoing permet avantageusement de protéger et renforcer les cheveux tout en traitant et réparant la fibre capillaire.

Un troisième test d'usage a été réalisé avec la composition cosmétique selon l'invention décrite dans l'exemple 5 ci-dessus et identifiée dans le tableau 12 comme composition N°1.

Pour ce faire, 37 femmes âgées entre 22 et 55 ans ont été recrutées pour cette étude afin de tester l'activité réparatrice, de la composition ainsi que les qualités « cosmétiques » de cette composition. De ce fait, la composition a été appliquée de deux à cinq fois par semaine pendant 28 jours dans les conditions normales d'utilisation.

Les caractéristiques des cheveux des femmes ayant participées au test sont résumés dans les tableaux 26 à 28 ci-dessous :

**Tableau 26 : qualité des cheveux en fonction des participants**

| Cheveux | Effectifs | pourcentage |
|---|---|---|
| Très secs | 6 | 16% |
| Secs | 21 | 41% |
| Normaux | 3 | 8% |
| Mixtes (racines grasses, pointes sèches) | 7 | 19% |
| Total | 37 | 100 % |

**Tableau 27 : longueur des cheveux en fonction des participants**

| Cheveux | Effectifs | pourcentage |
|---|---|---|
| Courts | 5 | 14% |
| Mi-longs | 24 | 65% |
| Longs | 8 | 22% |
| Total | 37 | 100% |

**Tableau 28: état des cheveux en fonction des participants**

| Cheveux | Effectifs | pourcentage |
|---|---|---|
| Très abîmés | 6 | 16% |
| Abîmés | 20 | 54% |
| Légèrement abîmés | 10 | 27% |
| En bon état | 1 | 3% |
| Total | 37 | 100% |

Le tableau 28 ci-dessous comprend les résultats obtenus et démontre l'efificacité de la composition utilisée.

| Propriétés | Favorable |
|---|---|
| A l'application | |
| Le packaging est pratique à utiliser : | 97% |
| La texture du masque est onctueuse : | 97% |
| La texture du masque est riche : | 97% |
| La texture du masque est soyeuse : | 97% |
| La texture du masque me plaît : | 97% |
| Ce masque démêle bien les cheveux : | 97% |
| Ce masque s'applique bien sur les cheveux : | 95% |
| Ce produit se rince facilement : | 92% |
| Ce masque procure un sentiment de bien-être : | 89% |
| On reconnaît bien le parfum des huiles essentielles : | 89% |
| Le parfum du masque est naturel : | 73% |
| Le parfum du masque est agréable : | 57% |
| Le parfum du masque est bien dosé : | 57% |

| Après utilisation | |
|---|---|
| Mes cheveux sont brillants : | 100% |
| Mes cheveux sont faciles à coiffer : | 100% |
| Mes cheveux sont plus résistants : | 97% |
| Mes cheveux restent "parfumés" : | 95% |
| Le masque laisse un fini agréable sur le cheveu : | 95% |
| Le masque aide à nourrir les cheveux : | 95% |
| Mes cheveux sont légers : | 95% |
| Mes cheveux sont soyeux : | 95% |
| Mes cheveux semblent réparés : | 95% |
| Mes cheveux sont faciles à démêler: | 95% |
| Mes cheveux sont protégés des agressions extérieures (sèche-cheveux, froid, soleil, brossage...) : | 95% |
| Mes cheveux semblent réparés jusqu'au bout de pointes : | 95% |
| Mes cheveux sont protégés : | 95% |
| Je n'ai pas d'électricité statique dans les cheveux : | 95% |
| Le produit est bien adapté aux cheveux secs, abîmés, fragilisés : | 95% |
| Mes cheveux sont hydratés : | 92% |
| Mes cheveux sont souples : | 92% |
| Mes cheveux sont plus forts : | 92% |
| La fibre de mes cheveux semble restaurée : | 92% |
| La fibre de mes cheveux semble réparée : | 92% |
| Mes cheveux semblent restructurés : | 92% |
| Mes cheveux sont moins fragiles : | 92% |
| Mes cheveux résistent mieux au brossage : | 92% |
| Mes cheveux ne sont pas alourdis : | 92% |
| Mes cheveux sont plus doux : | 89% |
| Les écailles de mes cheveux semblent lissées : | 89% |
| Les écailles de mes cheveux semblent scellées : | 89% |
| Mes cheveux sont gainés : | 89% |
| Le produit insuffle une nouvelle force aux cheveux : | 89% |
| Les brèches des cheveux semblent comblées : | 89% |
| Mes cheveux sont fortifiés : | 89% |
| Mes cheveux sont transformés : | 89% |
| Le démêlage est instantané : | 89% |
| Mes cheveux sont moins cassants : | 89% |
| Mes cheveux sont protégés de la casse : | 89% |
| Mes cheveux ont l'air éclatant de santé : | 89% |
| Mes cheveux résistent à la casse : | 89% |
| Mes cheveux offrent un toucher incroyablement doux : | 86% |
| J'ai moins de cheveux cassés au brossage : | 86% |
| Le produit a une efficacité anti-casse : | 86% |
| C'est un masque anti-casse : | 86% |
| J'ai moins de fourches : | 86% |
| Le masque ne laisse pas d'effet gras sur les cheveux : | 84% |
| Mes cheveux sont magnifiés : | 84% |

Tel que démontré dans cet exemple, une composition selon l'invention, ici un masque permet avantageusement de protéger et renforcer les cheveux tout en traitant et réparant la fibre capillaire.

### Exemple 8 : Etude in-vitro de l'effet de compositions selon l'invention par rapport à des compositions connues

Dans cet exemple, 10 mèches identiques de cheveux ont été choisies. Ils s'agissaient de collés trame dense de la société Euro-natural-hair d'une longueur de 20 centimètres, 18 centimètres de cheveux libres de couleur 6/0 avec une charge de 274. Ils ont été coupés en morceaux de 3 centimètres.

Sur 5 mèches, un shampoing neutre de l'état de la technique a été appliqué 6 fois. Entre chaque application la mèche a été rincée.

Sur les 5 autres mèches, la composition décrite dans l'exemple 3 identifiée composition N°1 dans le tableau 8 été appliquée puis rincée, ensuite la composition décrite dans l'exemple 4 identifiée composition N°1 dans le tableau 10 a été appliquée puis rincée, enfin la composition décrite dans l'exemple 5 identifiée composition N°1 dans le tableau 12 a été appliquée puis rincée. Ces étapes ont été répétées 6 fois pour chaque mèche. Les résultats ont été désignés sous la référence « association ».

Les mèches ont été alors soumises à un traitement avec un Spin Split Hair (marque déposée) pendant une heure sous un séchoir. Le dispositif était réglé à une vitesse de 75 tours par minute, représentant ainsi environ 9000 brossages par heures des mèches. Une étude de l'aspect des mèches a été ensuite réalisée selon le procédé suivant : chaque mèche a été divisée en 6 parts égales, représentant environ 50 cheveux par fibre. Une mesure et détermination du nombre de fourches par échantillon testé et du pourcentage nombre de fourches sur le nombre de fibres (50) ont été déterminées.

Par ailleurs une détermination du nombre de cheveux cassant été effectué. Pour ce faire, chaque mèche a été divisée en trois parts égales. Dix passages avec un peigne ont été réalisés sur un tiers de la mèche. Une collecte des cheveux cassants a été alors réalisée. La détermination du nombre de cheveux cassant par mèche a été ensuite effectuée.

Une étude sous microscopie électronique des mèches a été également réalisée avec un microscope électronique Zeiss DSM 982 Gemini.

Le tableau 29 ci-dessous représente les résultats du nombre de fourches en fonction de la composition appliquée et après traitement avec un Spin Split Hair (marque déposée) tel que décrit ci-dessus.

**Tableau 29 : nombre de fourches en fonction du traitement**

| | Shampoing de l'état de la technique | Association |
|---|---|---|
| Moyenne du nombre de fourches | 18,6 ±6,6 | 7,8±4 |
| Pourcentage du nombre de fourches en fonction du nombre de cheveux | 37,2%±12,2 | 15,5%±8,1 |

Tel que montré dans le tableau 29 ci-dessus, les compositions selon l'invention permettent de diminuer de 58,2% le nombre de fourches sur les cheveux.

Le tableau 30 ci-dessous représente le nombre de cheveux cassant en fonction de la composition appliquée.

| | Shampoing de l'état de la technique | Association |
|---|---|---|
| Moyenne du nombre de fourches | 33,5 ±6,6 | 5,6±5,4 |

Tel que montré dans le tableau 30 ci-dessus, les compositions selon l'invention permettent de diminuer de 83,1% le nombre de cheveux cassants.

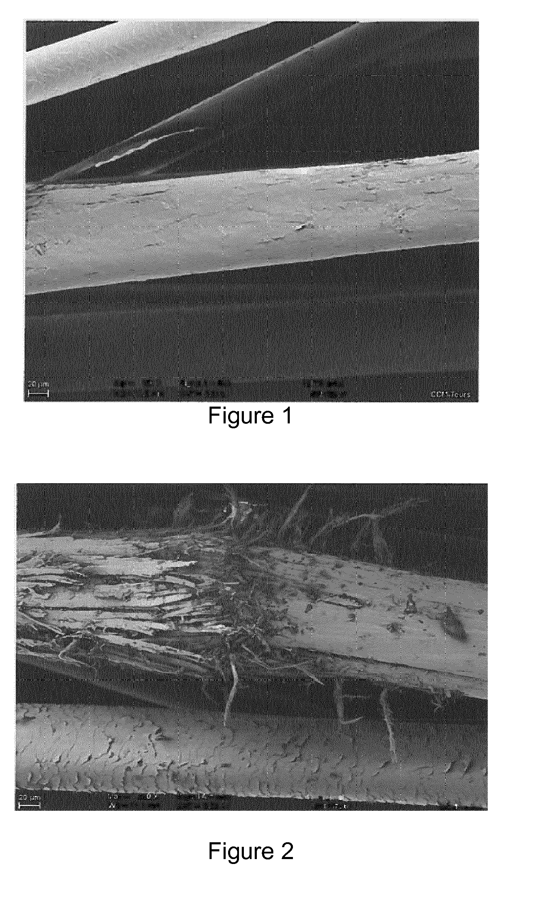

Enfin, la figure 1 représente une photographie d'une fibre capillaire en microscopie électronique après application de compositions selon l'invention telle que décrite ci-dessus. La figure 2 représente une photographie d'une fibre capillaire en microscopie électronique après application d'une composition de l'état de la technique. Tel que montré sur ces figures, il apparait clairement que la fibre sur laquelle une composition selon l'invention a été appliquée ne présente pas de cassure. Au contraire, la fibre sur laquelle a été appliqué une composition de l'état de la technique montre clairement une altération de la fibre, voir une rupture.

Cet exemple démontre donc clairement que la composition selon l'invention permet, notamment, de protéger les cheveux des agressions extérieures, en particulier permet de renforcer les fibres capillaires.

En outre, cet exemple démontre clairement que la composition selon l'invention permet de prévenir les fourches et les cassures de la fibre capillaire.

### BIBLIOGRAPHIE

1- Agache P. Présentation de la peau. In: Physiologie de la peau et explorations fonctionnelles cutanées. Cachan: EM Inter; 2000. p3-5.
2- THEVENIN Thierry, 2008. Le chemin des herbes. Les plantes sauvages, connaître, cueillir et utiliser. ED. Lucien Souny. 331 p.
3- Farid Chemat : Eco-extraction du vegetal, Editions Dunod, 2011.
4- Pongsavee M. In vitro study of lymphocyte antiproliferation and cytogenetic effect by occupational formaldehyde exposure. Toxicol Ind Health. 2011 Apr 19.
5- Seitz G, Bonin M, Fuchs J et al. Inhibition of glutathione-S-transferase as a treatment strategy for multidrug résistance in childhood rhabdomyosarcoma. International Journal of Oncology 2010 36:491-500.
6- Devilard E, Bladou F, Ramuz O, Karsenty G, Dalès JP, Gravis G, Nguyen C, Bertucci F, Xerri L, Birnbaum D. FGFR1 and WT1 are markers of human prostate cancer progression. BMC Cancer. 2006 Nov 30;6:272.
7- Archambaud C, Sansoni A, Mingueneau M, Devilard E, Delsol G, Malissen B, Malissen M. STAT6 deletion converts the Th2 inflammatory pathology afflicting Lat(Y136F) mice into a lymphoproliferative disorder involving Th1 and CD8 effector T cells. J Immunol. 2009 Mar 1;182(5):2680-9.
8- Versican, a major hyaluronan-binding component in the dermis, loses its hyaluronan-binding ability in solar elastosis. Hasegawa K, Yoneda M, Kuwabara H, Miyaishi O, Itano N, Ohno A, Zako M, Isogai Z. J Invest Dermatol. 2007 Jul; 127(7):1657-63.
9- Expression and purification of functionally active hyaluronan-binding domains from human cartilage link protein, aggrecan and versican: formation of ternary complexes with defined hyaluronan oligosaccharide. Seyfried NT, McVey GF, Almond A, Mahoney DJ, Dudhia J, Day AJ. J Biol Chem. 2005 Feb 18;280(7):5435-48.
10- Segmental versican expression in the trabecular meshwork and involvement in outflow facility. Keller KE, Bradley JM, Vranka JA, Acott TS. Invest Ophthalmol Vis Sci. 2011 Jul 7; 52(8):5049-57.
11- Impaired skin wound healing in lumican-null mice. Yeh JT, Yeh LK, Jung SM, Chang TJ, Wu HH, Shiu TF, Liu CY, Kao WW, Chu PH. Br J Dermatol. 2010 Dec;163(6): 1174-80.
12- A novel fibrotic disorder associated with increased dermal fibroblast proliferation and downregulation of genes of the microfibrillar network. Szauter KM, Ordas A, Laxer RM, Pope E, Wherrett D, Alman B, Mink M, Boyd CD, Csiszar K, Hinek A. Br J Dermatol. 2010 Nov; 163(5):1102-15.
13- Noblesse E, Cenizo V, Bouez C, Borel A, Gleyzal C, Peyrol S, Jacob MP, Sommer P, Damour O. Lysyl oxidase-like and lysyl oxidase are present in the dermis and epidermis of a skin equivalent and in human skin and are associated to elastic fibers. J Invest Dermatol. 2004 Mar;122(3):621-30.
14- Takaluoma K, Hyry M, Lantto J, Sormunen R, Bank RA, Kivirikko KI, Myllyharju J, Soininen R. Tissue-specific changes in the hydroxylysine content and cross-links of collagens and alterations in fibril morphology in lysyl hydroxylase 1 knock-out mice. J Biol Chem. 2007 Mar 2; 282(9):6588-96.
15- Lindner D, Zietsch C, Becher PM, Schulze K, Schultheiss HP, Tschöpe C, Westermann D. Differential expression of matrix metalloproteases in human fibroblasts with different origins. Biochem Res Int. 2012; 2012 : 875742.
16- Schweizer J, Bowden PE, Coulombe PA, Langbein L, Lane EB, Magin TM, Maltais L, Omary MB, Parry DA, Rogers MA, Wright MW. New consensus nomenclature for mammalian keratins. J Cell Biol. 2006 Jul 17;174(2):169-74.
17- Dong-Dong Wu, David M Irwin and Ya-Ping Zhang. Molecular evolution of the keratin associated protein gene family in mammals, role in the evolution of mammalian hair. BMC Evolutionary Biology 2008, 8:241.
18- Huber M, Siegenthaler G, Mirancea N, Marenholz I, Nizetic D, Breitkreutz D, Mischke D, Hohl D. Isolation and characterization of human repetin, a member of the fused gene family of the epidermal differentiation complex. J Invest Dermatol. 2005 May;124(5):998-1007.
19- Nakade K, Zheng H, Ganguli G, Buchwalter G, Gross C, Wasylyk B. The tumor suppressor p53 inhibits Net (ELK3), an effector of Ras/extracellular signal-regulated kinase signaling. Mol Cell Biol. 2004 Feb;24(3):1132-42.
20- Guadall A, Orriols M, Rodriguez-Calvo R, Calvayrac O, Crespo J, Aledo R, Martinez-Gonzàlez J, Rodriguez C. Fibulin-5 is up-regulated by hypoxia in endothelial cells through a hypoxia-inducible factor-1 (HIF-1α)-dependent mechanism. J Biol Chem. 2011 Mar 4;286(9):7093-103.
21- Preis M, Cohen T, Sarnatzki Y, Ben Yosef Y, Schneiderman J, Gluzman Z, Koren B, Lewis BS, Shaul Y, Flugelman MY. Effects of fibulin-5 on attachment, adhesion, and proliferation of primary human endothelial cells.Biochem Biophys Res Commun. 2006 Sep 29; 348(3):1024-33.
22- Perdiguero EG, Galaup A, Durand M, Teillon J, Philippe J, Valenzuela DM, Murphy AJ, Yancopoulos GD, Thurston G, Germain S. Alteration of developmental and pathological retinal angiogenesis in angptl4-deficient mice. J Biol Chem. 2011 Oct 21; 286(42):36841-51.

## Revendications

1. Composition cosmétique comprenant au moins un extrait de racines d'angélique et un extrait de graines d'angélique.

2. Composition selon la revendication 1, dans laquelle l'extrait de racines d'angélique est choisi parmi un extrait aqueux, un extrait huileux, une huile essentielle ou un mélange de ceux-ci.

3. Composition selon la revendication 1 ou 2, dans laquelle l'extrait de graines d'angélique est choisi parmi un extrait hydro-alcoolique glycériné, un extrait huileux, une huile essentielle ou un mélange de ceux-ci.

4. Composition selon l'une quelconque des revendications 1 à 3 dans laquelle la composition comprend une huile essentielle de racines d'angélique, un extrait aqueux et/ou huileux de racines d'angélique et un extrait de graines d'angélique.

5. Composition selon l'une quelconque des revendications 1 à 4 dans laquelle l'extrait de racines d'angélique est une huile essentielle de racines d'angélique et l'extrait de graines d'angélique est une huile essentielle de graines d'angélique.

6. Composition selon l'une quelconque des revendications 1 à 5 dans laquelle la concentration d'extrait de racines d'angélique est de 0,001% à 10% en poids par rapport au poids total de la composition.

7. Composition selon l'une quelconque des revendications 1 à 6 dans laquelle la concentration d'extrait de graines d'angélique est de 0,001% à 10% en poids par rapport au poids total de la composition.

8. Composition selon l'une quelconque des revendications précédentes, ladite composition étant sous une forme choisie parmi une crème, un lait, un gel, une lotion, un onguent, un patch, un shampooing, un après-shampooing, un masque, un sérum.

9. Utilisation cosmétique d'une composition selon l'une quelconque des revendications 1 à 8.

10. Utilisation cosmétique d'une composition selon la revendication 4 pour l'hydratation cutanée.

11. Utilisation cosmétique d'une composition selon la revendication 4 pour le traitement du vieillissement cutanée.

12. Utilisation cosmétique d'une composition selon l'une quelconque des revendications 1 à 8 pour le traitement de la casse et/ou altération des fibres capillaires.

13. Utilisation cosmétique d'une composition selon l'une quelconque des revendications 1 à 8 pour le traitement de la casse et/ou la protection du cheveu et/ou de la fibre capillaire des agressions extérieures et/ou stimulation de la pousse du cheveu, le renforcement des cheveux et/ou le traitement cosmétique de la chute des cheveux.

14. Procédé de traitement cosmétique non thérapeutique comprenant l'application sur la peau et/ou sur les cheveux d'une composition cosmétique selon l'une quelconque des revendications revendication 1 à 8.

15. Procédé de traitement cosmétique non thérapeutique pour la l'hydratation cutanée en surface comme en profondeur, la stimulation de la pousse, le renforcement des cheveux et/ou le traitement cosmétique de la chute des cheveux comprenant l'application sur la peau et/ou sur les cheveux d'une composition cosmétique selon l'une quelconque des revendications 1 à 8.

16. Procédé de soin cosmétique anti-âge comprenant une étape d'application sur la peau d'une composition cosmétique telle que définie dans la revendication 4.

17. Procédé de soin cosmétique hydratant comprenant une étape d'application sur la peau d'une composition cosmétique telle que définie dans la revendication 4.

18. Procédé de soin cosmétique pour la réparation capillaire comprenant une étape d'application sur le cuir chevelu et/ou les cheveux d'une composition cosmétique telle que définie dans l'une quelconque des revendications 1 à 8.
